# EUROPEAN PATENT SPECIFICATION

(11) **EP 4 468 997 B1**
(45) Date of publication and mention of the grant of the patent: **04.11.2026**
(21) Application number: 23702672.9
(22) Date of filing: 20.01.2023
(51) Int. Cl.: A61F 2/95, A61F 2/24

(54) **HYDRAULIC DEPLOYMENT AND RECAPTURE SYSTEM FOR A PROSTHETIC HEART VALVE**
HYDRAULISCHES EINSATZ- UND WIEDERAUFNAHMESYSTEM FÜR EINE HERZKLAPPENPROTHESE
SYSTÈME DE DÉPLOIEMENT ET DE RECAPTURE HYDRAULIQUE POUR UNE VALVULE PROTHÉTIQUE

(30) Priority: 28.01.2022 US 202263304033 P
(43) Date of publication of application: 04.12.2024
(73) Proprietor: Medtronic, Inc., Minneapolis, MN 55432 (US)
(72) Inventor: GRISWOLD, Erik, Santa Rosa, California 95403 (US); BRUNER, Kenny, Santa Rosa, California 95403 (US); ADAIR, Caralin, Santa Rosa, California 95407 (US); SIMPSON, Mike, Santa Rosa, California 95403 (US)
(74) Representative: Zimmermann & Partner Patentanwälte mbB
(86) International application number: PCT/IB2023/050495
(87) International publication number: WO 2023/144670

(56) References cited:
- EP-A2- 4 119 096
- US-A1- 2013 304 200
- US-A1- 2022 008 194

## Description

### FIELD OF THE INVENTION

The present technology is generally related to prosthetic valve delivery devices.

### BACKGROUND OF THE INVENTION

Patients suffering from various medical conditions or diseases may require surgery to install an implantable medical device. For example, valve regurgitation or stenotic calcification of leaflets of a heart valve may be treated with a heart valve replacement procedure. A traditional surgical valve replacement procedure requires a sternotomy and a cardiopulmonary bypass, which creates significant patient trauma and discomfort. Traditional surgical valve procedures may also require extensive recuperation times and may result in life-threatening complications.

One alternative to a traditional surgical valve replacement procedure is delivering implantable medical devices using minimally-invasive techniques. For example, a prosthetic valve can be percutaneously and transluminally delivered to an implant location. In such methods, the prosthetic valve can be compressed within a delivery catheter for insertion within a patient's vasculature and once inserted may be expanded at a target location. Among devices commonly used to access vascular and other locations within a body and to perform various functions at those locations are medical catheter, or delivery catheters, adapted to deliver and deploy medical devices such as prosthetic valves, stent-grafts, and stents to selected targeted sites in the body. Such medical devices typically are releasably carried within a distal region of the delivery catheter in a radially compressed delivery configuration as the catheter is navigated to and positioned at a target treatment/deployment site.

Typically, a delivery catheter will deploy the prosthetic valve using a mechanical or hydraulic systems and the forces created by these systems will expel the prosthetic valve into the target treatment site. During the delivery process, it is possible for the delivery catheter to experience non-uniform forces during the deployment process due to friction or inconsistencies within the mechanical or hydraulic systems. These non-uniform forces may lead to jumping of the prosthetic valve in the catheter, inability to deploy the prosthetic valve, and/or inadvertent deployment of the valve. Therefore, a need exists for improved delivery devices configured to provide a consistent and reliable delivery process within a patient's anatomy.

US 2013/0304200 A1 describes prosthetic heart valve devices and prosthetic mitral valves.

### BRIEF SUMMARY OF THE INVENTION

According to a first embodiment hereof, corresponding to the invention, a system is provided for deploying a valve prosthesis which includes a deployment device configured to deploy a valve prosthesis. The deployment device including an elongated catheter body having a distal end and a proximal end, the catheter body further including a lumen extending a length of the catheter body. A tension cable having a first end and a second end, the tension cable positioned within the catheter body. A capsule having a delivery configuration and a deployed configuration, the capsule positioned at the distal end of the catheter body and operatively coupled to the lumen and the first end of the tension cable, wherein the tension cable inhibits the capsule from transitioning between the delivery configuration and the deployed configuration. A handle positioned at the proximal end of the catheter body and operatively coupled to the second end of the tension cable and the lumen. A deployment assist positioned at the handle and is configured to place a force on the second end of the tension cable in order to assist in transitioning the capsule from the delivery configuration to the deployed configuration, wherein the lumen and the capsule are configured to hold a hydraulic fluid. A hydraulic system fluidly connected to the capsule and the lumen, wherein the hydraulic system is configured to transition the capsule from the delivery configuration to the deployed configuration via delivery of the hydraulic fluid into the capsule through the lumen.

In an aspect of the first embodiment, and in combination with any other aspects herein, the disclosure provides that the deployment assist is rotatable relative to the handle, wherein the rotational movement of the deployment assist places the force on the second end of the tension cable, wherein the force is an axial force in a distal direction

In an aspect of the first embodiment, the disclosure provides that the capsule further includes a body and a deployment piston, wherein transitioning the capsule from the delivery configuration to the deployed configuration moves the body axially relative to the deployment piston.

In an aspect of the first embodiment, the disclosure provides that the capsule further includes a deployment chamber between the body and the deployment piston and fluidly coupled to the lumen, the deployment chamber is configured to fill with hydraulic fluid to transition the capsule from the delivery configuration to the deployed configuration.

In an aspect of the first embodiment, the disclosure provides that the first end of the tension cable is coupled to the body of the capsule, the first end of the tension cable being configured to place an axial force on the body of capsule as to move the body of the capsule axially relative to the deployment piston.

In an aspect of the first embodiment, the disclosure provides that the deployment assist is a cap that is positioned radially over the proximal end of the handle, the cap is configured to have a plurality of threads that convert rotational movement of the cap into the force that is placed on the second end of the tension cable.

In an aspect of the first embodiment, the disclosure provides that the deployment assist further includes a locking aperture, the locking aperture configured to receive a pin that secures the cap to the handle.

In an aspect of the first embodiment, the disclosure provides that the deployment assist is a plunger that is positioned within the proximal end of the handle, the plunger extends radially from the distal end of the handle and is configured to have a plurality of threads that convert the rotational movement of the plunger into the force that is placed on the second end of the tension cable.

In an aspect of the first embodiment, the disclosure provides that the deployment assist further includes a lever including a cam that is configured to place the force onto the second end of the tension cable as the lever is rotated.

In an aspect of the first embodiment, the disclosure provides that the system further includes a distal push assist at the capsule, the distal push assist being configured to assist in transitioning the capsule between the delivery and deployed configurations. The distal push assist may be a spring.

According to a second embodiment hereof, the disclosure provides a method for deploying a valve prosthesis including positioning a deployment device at a target location. The deployment device including an elongated catheter body having a distal end and a proximal end, the catheter body further including a lumen extending a length of the catheter body. A tension cable having a first end and a second end, the tension cable positioned within the catheter body. A capsule having a delivery configuration and a deployed configuration, the capsule positioned at the distal end of the catheter body and operatively coupled to the lumen and the first end of the tension cable, wherein the tension cable inhibits the capsule from transitioning between the delivery configuration and the deployed configuration. A handle positioned at the proximal end of the catheter body and operatively coupled to the second end of the tension cable and the lumen. A deployment assist positioned at the handle, wherein the lumen and the capsule are configured to hold hydraulic fluid. Once positioned, the method includes increasing a pressure of the hydraulic fluid within the lumen and the capsule using a hydraulic system; applying a first force to the second end of the tension cable using the deployment assist; and transitioning the capsule between the delivery configuration to the deployed configuration.

In an aspect of the second embodiment, the disclosure provides that applying the force to the second end of the tension cable using the deployment assist begins the step of transitioning the capsule between the delivery configuration to the deployed configuration.

In an aspect of the second embodiment, the disclosure provides that the pressure of the hydraulic fluid places a second force onto the capsule.

In an aspect of the second embodiment, the disclosure provides that the method further includes transitioning the capsule from the deployed configuration to the delivery configuration and removing the deployment device from the target location.

In an aspect of the second embodiment, the disclosure provides that the capsule further includes a body and a deployment piston.

In an aspect of the second embodiment, the disclosure provides that transitioning the capsule from the delivery configuration to the deployed configuration further includes moving the body axially relative to the deployment piston.

According to a third embodiment hereof, the disclosure provides a system for deploying a valve prosthesis, the system including a deployment device configured to deploy a valve prosthesis. The deployment device includes an elongated catheter body having a distal end and a proximal, the catheter body further including a lumen extending a length of the catheter body. A tension cable having a first end and a second end, the tension cable positioned within the catheter body. A capsule having a delivery configuration and a deployed configuration, the capsule positioned at the distal end of the catheter body and operatively coupled to the lumen and the first end of the tension cable, wherein the tension cable inhibits the capsule from transitioning between the delivery configuration and the deployed configuration. A handle positioned at the proximal end of the catheter body and operatively coupled to the tension cable and the lumen, wherein the lumen and the capsule are configured to hold a hydraulic fluid. The system further includes a hydraulic system configured to transition the capsule from the delivery configuration to the deployed configuration. The hydraulic system includes a primary fluid cylinder fluidly connected to the lumen and the capsule, the primary fluid cylinder being configured to increase the pressure of the hydraulic system to a specified pressure level and a secondary fluid cylinder fluidly connected to the lumen and the capsule, the secondary fluid cylinder being configured to supplement fluid volumes within the lumen and the capsule.

In an aspect of the third embodiment, the disclosure provides that the first end of the tension cable exerts a first axial force in a first direction on the capsule.

In an aspect of the third embodiment, the disclosure provides that the pressure within the lumen and the capsule exerts a second axial force in a second direction on the capsule, the second direction being opposite the first direction.

In an aspect of the third embodiment, and in combination with any other aspects herein, the disclosure provides that the capsule transitions from the delivery configuration to the deployed configuration when the second axial force is larger than the first axial force.

In an aspect of the third embodiment, the disclosure provides that the system further includes a check valve configured to control how much fluid volume is supplemented by the secondary cylinder.

According to a fourth embodiment hereof, the disclosure provides a method for deploying a valve prosthesis including positioning a deployment device at a target location. The deployment device including an elongated catheter body having a distal end and a proximal end, the catheter body further including a lumen extending a length of the catheter body. A tension cable having a first end and a second end, the tension cable positioned within the catheter body. A capsule having a delivery configuration and a deployed configuration, the capsule positioned at the distal end of the catheter body and operatively coupled to the lumen and the first end of the tension cable, wherein the tension cable inhibits the capsule from transitioning between the delivery configuration and the deployed configuration. A handle positioned at the proximal end of the catheter body and operatively coupled to the second end of the tension cable and the lumen. A deployment assist positioned at the handle, wherein the lumen and the capsule are configured to hold hydraulic fluid. The method further includes increasing the pressure of the hydraulic fluid within the lumen and the capsule using a hydraulic system, the hydraulic system being fluidly connected to the capsule and the lumen. The hydraulic system includes a primary fluid cylinder and a second fluid cylinder fluidly connected to the lumen and the capsule. The method further includes supplementing the fluid volumes within the lumen and the capsule with the secondary cylinder.

In an aspect of the fourth embodiment, the disclosure provides that the method further includes transitioning the capsule between the delivery configuration and the deployed configuration when the pressure of the hydraulic fluid applies a first force to the capsule that is greater than a second force applied to the capsule by the tension cable.

In an aspect of the fourth embodiment, the disclosure provides that the method further includes maintaining the fluid volumes within the lumen and the capsule with the secondary cylinder as to maintain the pressure of the hydraulic fluid as the capsule transitions between the delivery configuration and the deployed configuration.

In an aspect of the fourth embodiment, the disclosure provides that supplementing the fluid volumes within the lumen and the capsule with the secondary cylinder is configured to maintain the pressure of the hydraulic fluid as to keep the first force applied to the capsule being greater than the second force being applied to the capsule by the tension cable.

In an aspect of the fourth embodiment, the disclosure provides that the method further includes transitioning the capsule from the deployed configuration to the delivery configuration and removing the deployment device from the target location.

### BRIEF DESCRIPTION OF DRAWINGS

The foregoing and other features and advantages of the invention will be apparent from the following description of embodiments thereof as illustrated in the accompanying drawings. The accompanying drawings, which are incorporated herein and form a part of the specification, further serve to explain the principles of the invention and to enable a person skilled in the art to make and use the invention. The drawings are not to scale.
FIG. 1 depicts a perspective view of a prosthetic heart valve in accordance with an aspect of the disclosure.
FIG. 2 depicts an atrial end view of the prosthetic heart valve shown in FIG. 1 in accordance with an aspect of the disclosure.
FIG. 3 depicts a side view of a delivery device according to an embodiment hereof, wherein the delivery device is configured for delivering the prosthetic heart valve of FIG. 1 within a capsule of the delivery device.
FIG. 4 depicts a side sectional view of the delivery device of FIG. 3, the delivery device being fluidly coupled to a hydraulic system and a deployment assist being disposed over a proximal end of the delivery device.
FIG. 5 is a cross-sectional view taken along line A-A of FIG. 3.
FIG. 6 is an exploded view of the delivery device of FIG. 3.
FIG. 7A is an enlarged view of a distal portion of an innermost shaft assembly of the delivery device of FIG. 3, wherein the innermost shaft assembly includes a flexible shaft, a piston mount, a piston, a tension cable, a distal shaft, a capsule, and a capsule cap.
FIG. 7B is an enlarged view of a first subassembly of the inner most shaft assembly of FIG. 7A, wherein the first subassembly includes the flexible shaft, the piston mount and the deployment piston.
FIG. 7C is an enlarged view of a second subassembly of the innermost shaft assembly of FIG. 7A, wherein the second subassembly includes the tension cable, the distal shaft, the capsule, and the capsule cap.
FIG. 7D is a sectional view of the distal shaft and the capsule cap of FIG. 7C.
FIG. 7E is an enlarged view of a distal portion of the shaft assembly with a distal push assist according to another embodiment hereof.
FIG. 8 is a perspective view of the deployment piston of the delivery device of FIG. 3, wherein the deployment piston is shown removed from the delivery device for sake of illustration only.
FIG. 9 is a side view of a distal portion of the delivery device of FIG. 3, wherein the distal portion includes the capsule and the deployment piston, the capsule being shown in a first position relative to the deployment piston and the prosthetic heart valve of FIG. 1 being loaded into the capsule.
FIG. 10 is a side view of the distal portion of FIG. 9, the capsule being shown in a second position relative to the deployment piston and the prosthetic heart valve of FIG. 1 being omitted for sake of clarity.
FIG. 11 is a side view of the distal portion of FIG. 9, the capsule being shown in a third position relative to the deployment piston and the prosthetic heart valve of FIG. 1 being omitted for sake of clarity.
FIG. 12 depicts a sectional view of a manifold of the delivery device of FIG. 3, the delivery device being in a delivery configuration.
FIG. 12A depicts a perspective view of a portion of FIG. 12.
FIG. 13 depicts a sectional view of the manifold of the delivery device of FIG. 3, the delivery device being in a deployed configuration.
FIG. 14 depicts an enlarged perspective view of a proximal portion of the delivery device of FIG. 3, wherein the deployment assist is disposed over the proximal end of the manifold of the delivery device.
FIG. 15 depicts an enlarged perspective view of the proximal portion of the delivery device of FIG. 3, wherein the deployment assist is disposed over the proximal end of the manifold and is shown in phantom.
FIG. 16 depicts a sectional view of a deployment assist according to another embodiment hereof incorporated into the delivery device of FIG. 3, the deployment assist including a threaded shaft and a release mechanism.
FIG. 16A depicts a perspective view of an exemplary embodiment of the release mechanism of FIG. 16.
FIG. 16B depicts a sectional view of the release mechanism of FIG. 16B.
FIG. 17A illustrates a deployment assist according to another embodiment hereof incorporated into the delivery device of FIG. 3, the deployment assist including a lever including a cam surface and the lever being shown in a first position.
FIG. 17B illustrates the deployment assist of FIG. 17A, the lever being shown in a second position.
FIG. 17C illustrates another embodiment of a deployment assist according to another embodiment hereof incorporated into the delivery device of FIG. 3, the deployment assist including a lever including a cam surface and the lever being shown in a first position, the deployment assist also being configured to lock in an initial position.
FIG. 17D illustrates the alternative embodiment of the deployment assist of FIG. 17C, the lever being shown in a second position.
FIG. 18 illustrates a hydraulic control mechanism according to an embodiment hereof, the hydraulic control mechanism being incorporated into the delivery device of FIG. 3 and being configured to dampen or mitigate forces during deployment of the prosthetic heart valve.
FIG. 19 illustrates a hydraulic deployment assist according to an embodiment hereof incorporated into the delivery device of FIG. 3.

### DETAILED DESCRIPTION OF THE INVENTION

Specific embodiments of the present invention are now described with reference to the figures, wherein like reference numbers indicate identical or functionally similar elements. The terms "distal" and "proximal" when used in the following description to refer to a delivery device or catheter are with respect to a position or direction relative to the treating clinician. Thus, "distal" and "distally" refer to positioned distant from, or in a direction away from the treating clinician, and the terms "proximal" and "proximally" refer to positions near, or in a direction toward the clinician.

The following detailed description is merely exemplary in nature and is not intended to limit the invention or the application and use of the invention. Although the description of the invention is in the context of the treatment and navigation of a mitral or tricuspid heart valve, the invention may be used where it is deemed useful in other anatomical sites that are not in the heart. For example, the present invention may be applied to other heart valves or venous valves as well. Furthermore, there is no intention to be bound by any expressed or implied theory presented in the preceding technical field, background, brief summary, or the following detailed description.

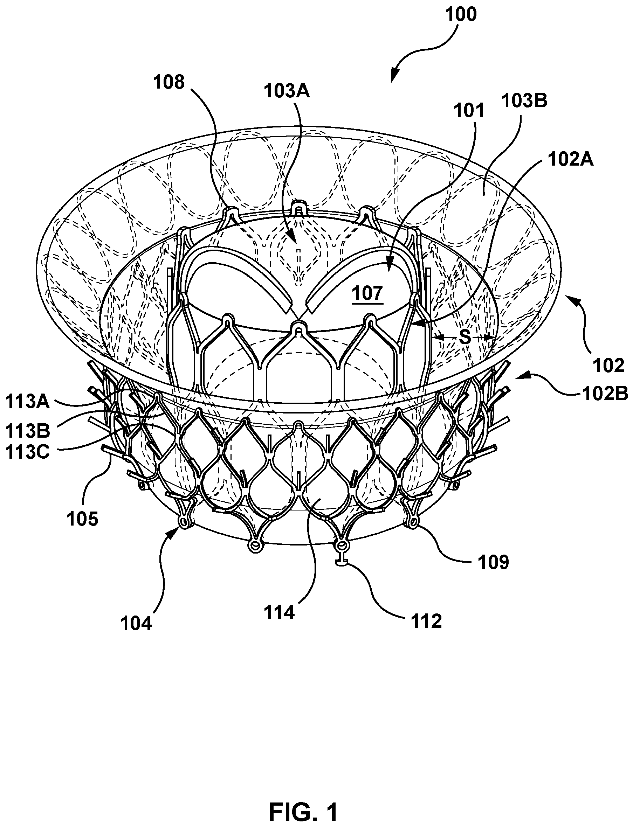

FIG. 1 illustrates an exemplary prosthetic heart valve 100 for use in embodiments hereof. Prosthetic heart valve 100 is illustrated herein in order to facilitate description of the interaction between the prosthetic heart valve 100 and a delivery device to be utilized in conjunction therewith according to embodiments hereof. It is understood that any number of alternate heart valve prostheses can be used with the methods and devices described herein. The prosthetic heart valve 100 is presented by way of example only, and other shapes and designs of prosthetic heart valves are also consistent with embodiments hereof. Other non-limiting examples of prosthetic heart valves that can be delivered via the delivery devices and methods described herein are described in U.S. Appl. No. 16/853,851 to McVeigh et al., U.S. Patent No. 9,034,032 to McLean et al. and International Patent Application No. PCT/US5114/029549 to McLean et al. Although the prosthetic heart valve 100 is configured for placement within a tricuspid heart valve or a mitral heart valve, embodiments of delivery devices and techniques described herein may be used in conjunction with any transcatheter valve prostheses. For example, embodiments described herein may be utilized with a transcatheter prosthetic heart valve configured for placement within a pulmonary, aortic, mitral, or tricuspid valve. There is no intention to be bound by any expressed or implied theory presented in the preceding technical field, background, brief summary or the following detailed description.

The prosthetic heart valve 100 is configured to be radially compressed into a reduced-diameter configuration (not shown) for delivery within a vasculature and to return to an expanded, deployed configuration, which is shown in FIG 1. Stated another way, the prosthetic heart valve 100 has a crimped configuration for delivery within a vasculature and an expanded configuration for deployment within a native heart valve. In accordance with embodiments hereof, when in the radially compressed or reduced-diameter configuration, the prosthetic heart valve 100 has a low profile suitable for delivery to and deployment within a native heart valve via a suitable delivery device that may be tracked to the deployment site of the native heart valve of a heart via any one of a transatrial, antegrade, or transapical approach. The prosthetic heart valve 100 includes a stent or frame 102 and a valve component 101 including at least one leaflet 107 disposed within and secured to the frame 102. The valve component 101 of the prosthetic heart valve 100 is capable of regulating flow therethrough via valve leaflets that may form a replacement valve.

Any portion of the frame 102 described herein as an element of the prosthetic heart valve 100 may be made from any number of suitable biocompatible materials, e.g., stainless steel, nickel titanium alloys such as Nitinol^{™}, cobalt chromium alloys such as MP35N, other alloys such as ELGILOY^{®} (Elgin, Ill.), various polymers, pyrolytic carbon, silicone, polytetrafluoroethylene (PTFE), or any number of other materials or combination of materials. A suitable biocompatible material would be selected to provide the prosthetic heart valve 100 to be configured to be compressed into a reduced-diameter crimped configuration for transcatheter delivery to a native valve, whereby release from a delivery catheter returns the prosthesis to an expanded, deployed configuration. Alternatively, the prosthetic heart valve 100 may be balloon-expandable as would be understood by one of ordinary skill in the art.

In an aspect of the disclosure, the frame 102 of the prosthetic heart valve 100 includes a valve support 102A at least partially surrounded by and attached to an anchoring member 102B. The valve support 102A is configured to support the valve component 101 therein. The valve support 102A is a tubular stent-like or frame structure that defines a central lumen from a first end 108 of the valve support 102A to a second end 109 of the valve support 102A. When positioned in situ within a native mitral valve, the first end 108 is an inflow or upstream end and the second end 109 is an outflow or downstream end. At the second end 109, the valve support 102A is attached to the anchoring member 102B via a plurality of connector components 104. In an embodiment, the plurality of connector components are rivets. In addition, at the second end 109, the valve support 102A also includes a plurality of attachment bars 112 extending therefrom that function to releasably couple the prosthetic heart valve 100 to a delivery device.

The anchoring member 102B is a stent-like or frame structure that functions as an anchor for the prosthetic heart valve 100 to secure its deployed position within a native annulus. The anchoring member 102B is a substantially cylindrically-shaped structure that is configured to engage heart tissue at or below an annulus of a native heart valve, such as an annulus of a native mitral valve. At the first end 108 of the valve support 102A, the anchoring member 102B is radially spaced a distance S from the valve support 102A to mechanically isolate the inflow end 108 of the valve support 102A from the anchoring member 102B. The anchoring member 102B includes one or more fixation elements 105 that extend outward from an exterior side thereof to engage heart tissue. The fixation elements 105 project radially outward and are inclined toward an upstream direction. The fixation elements 105, for example, can be prongs, cleats, barbs, hooks, or other elements that are inclined only in the upstream direction (e.g., a direction extending away from the downstream portion of the prosthetic heart valve 100.

The anchoring member 102B includes a plurality of crowns 113A and a plurality of struts 113B with each crown 113A being formed between a pair of opposing struts 113B. Each crown 113A is a curved segment or bend extending between opposing struts 113B. The anchoring member 102B is tubular, with a plurality of side openings 114 being defined by edges of the plurality of crowns 113A and the plurality of struts 113B. In an embodiment, the plurality of side openings 114 may be substantially diamond-shaped. The anchoring member 102B includes a plurality of nodes 113C. A node 113C is defined as a region where two crowns of the plurality of crowns 113A within the anchoring member 102B meet or connect. When attached to the valve support 102A via the plurality of connecting components 104, the anchoring member 102B forms an outer frame portion of the frame 102 and the valve support 102A forms an inner frame portion of the frame 102 with the anchoring member 102B circumferentially surrounding the valve support 102 disposed therein.

Each of the valve support 102A and the anchoring member 102B include a skirt or graft material 103A, 103B, respectively, secured thereto. More particularly, the graft material 103A is coupled to an inner surface of the valve support 102A to line a portion thereof. Alternatively, the graft material 103A may be coupled to an outer surface of the valve support 102A to enclose a portion thereof as would be known to one of ordinary skill in the art of prosthetic valve construction. The graft material 103B is coupled to an inner surface of the anchoring member 102B to line a portion thereof. The outer engagement surface of the anchoring member 102 is not covered any sealing or graft material so that the outer engagement surface directly contacts the tissue of the native annulus. The graft material 103A, 103B may be a natural or biological material such as pericardium or another membranous tissue such as intestinal submucosa. Alternatively, the graft material 103A, 103B may be a low-porosity woven fabric, such as polyester, Dacron fabric, or PTFE, which creates a one-way fluid passage when attached to the stent.

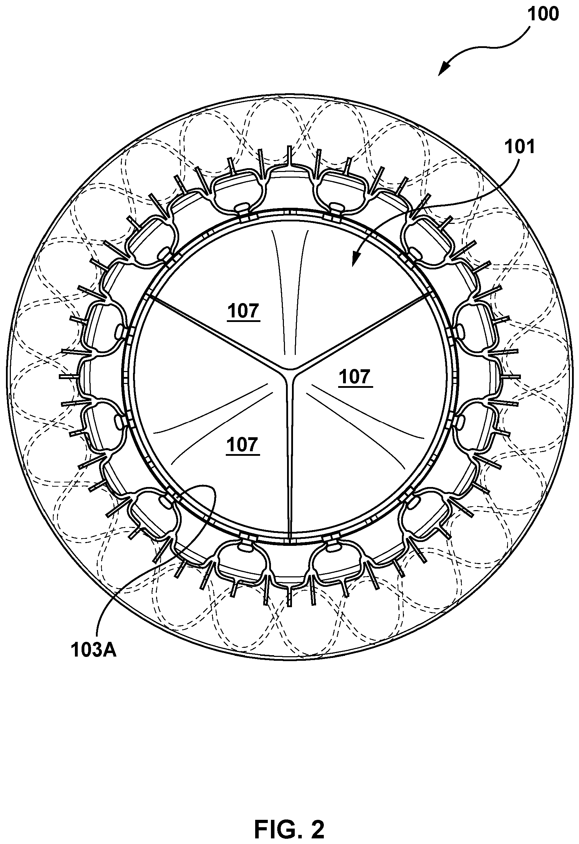

The valve component 101 of the prosthetic heart valve 100 is capable of regulating flow therethrough via valve leaflets 107 that may form a replacement valve. FIGS. 1 and 2 illustrates an exemplary valve component having three leaflets, although a bicuspid leaflet configuration may alternatively be used in embodiments hereof. When deployed in situ, the valve component 101 in a closed state is configured to block blood flow in one direction to regulate blood flow through the central lumen of the valve support 102A. The valve component 101 includes valve leaflets 107, e.g., three valve leaflets 107, that are disposed to coapt within an upstream portion of the valve support 102A with leaflet commissures of the valve leaflets 107 being secured within a downstream portion of the valve support 102A, such that the valve leaflets 107 open during diastole. Leaflets 107 are attached along their bases to the valve support 102A, for example, using sutures or a suitable biocompatible adhesive. Adjoining pairs of leaflets 107 are attached to one another at their lateral ends to form leaflet commissures. The orientation of the leaflets 107 within the valve support 102A depends upon on which end of the prosthetic heart valve 100 is the inflow end and which end of the prosthetic heart valve 100 is the outflow end, thereby ensuring one-way flow of blood through the prosthetic heart valve 100.

The valve leaflets 107 are attached to the graft material 103A in order to form the valve component 101. The valve leaflets 107 may be formed of various flexible materials including, but not limited to natural pericardial material such as tissue from bovine, equine or porcine origins, or synthetic materials such as polytetrafluoroethylene (PTFE), DACRON^{®} polyester, pyrolytic carbon, or other biocompatible materials. With certain prosthetic leaflet materials, it may be desirable to coat one or both sides of the replacement valve leaflet with a material that will prevent or minimize overgrowth. It is further desirable that the prosthetic leaflet material is durable and not subject to stretching, deforming, or fatigue.

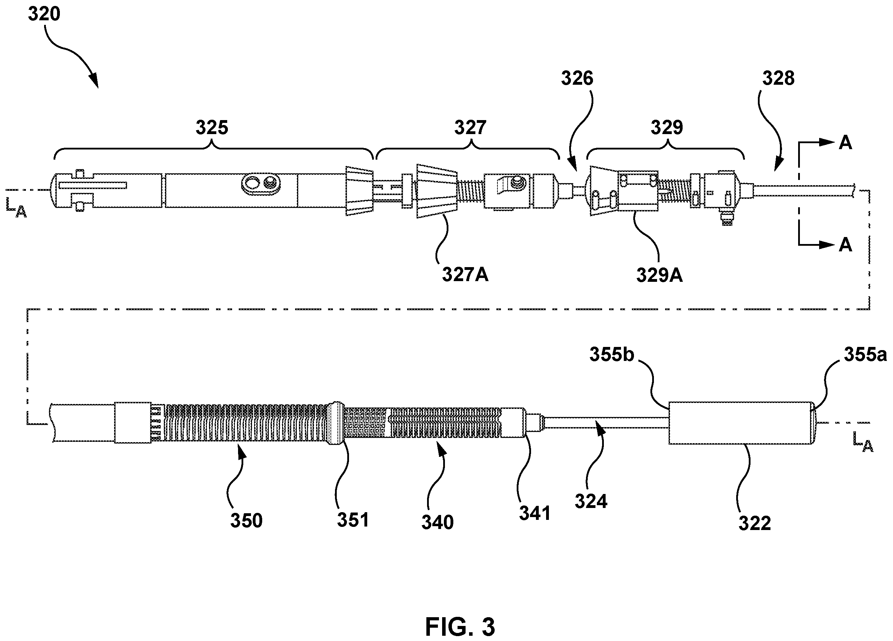

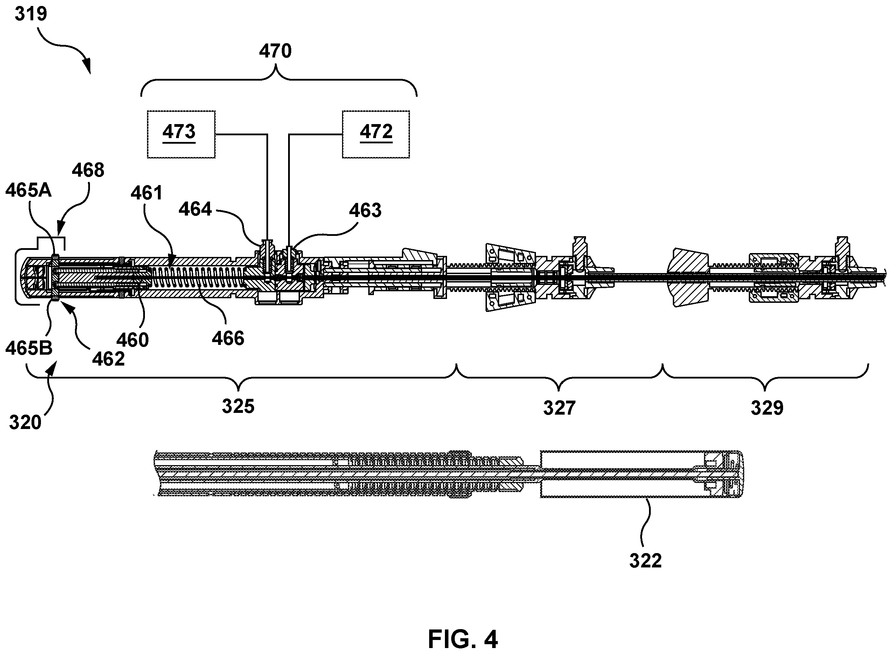

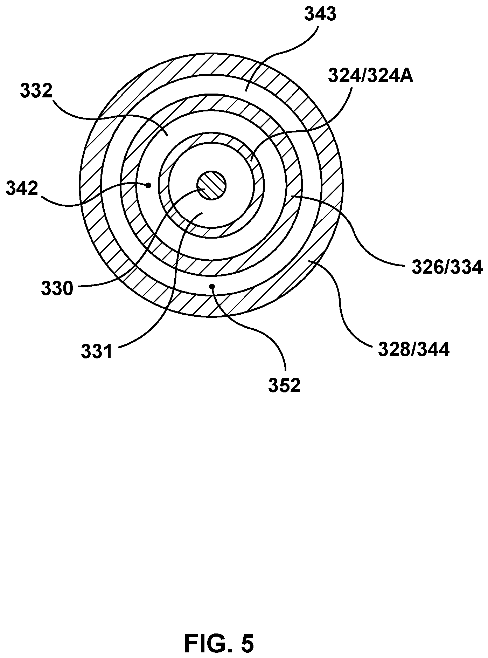

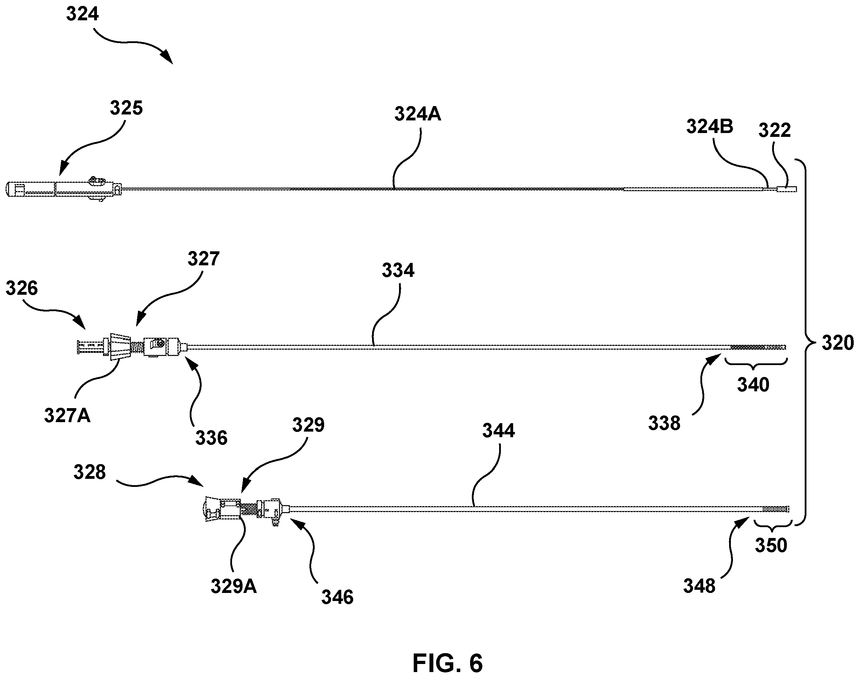

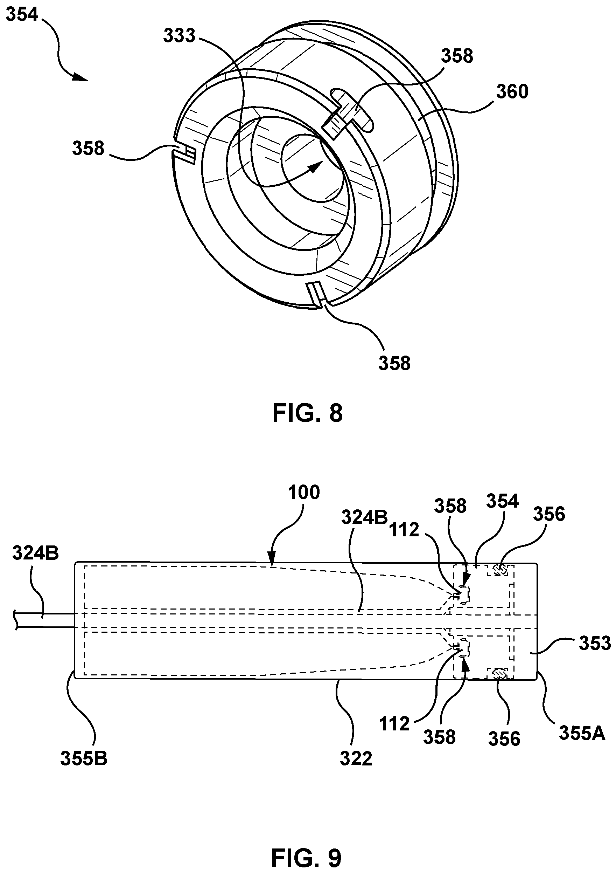

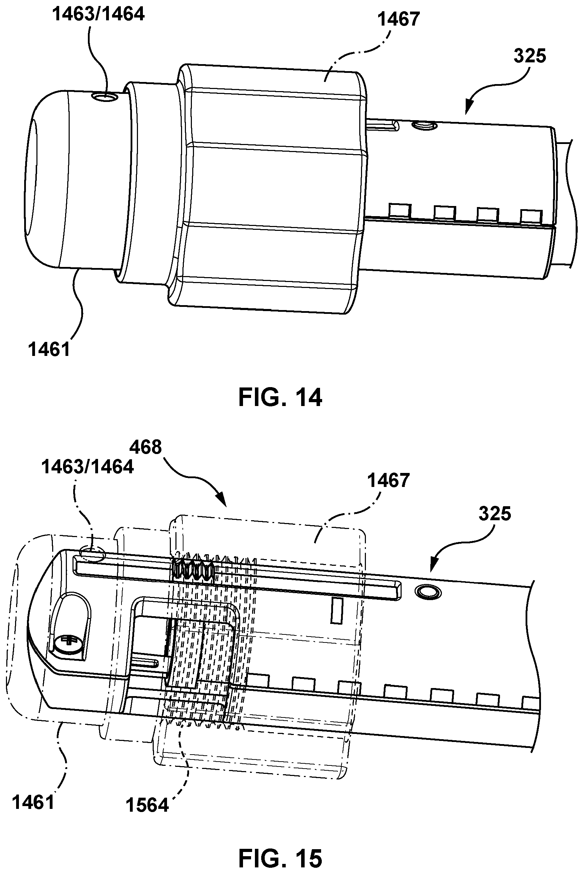

A delivery device 320 which may be used for transcatheter delivery and deployment of an implant, such as the non-limiting example of the prosthetic heart valve 100 of FIGS. 1 and 2, is shown in FIGS. 3-8. In general terms, the delivery device 320 is arranged and configured for percutaneously delivering an implant (e.g., prosthetic heart valve 100) in a delivery configuration to a patient's native defective heart valve or other portion of a patient's anatomy via transcatheter delivery. FIG. 3 illustrates a side view of the delivery device 320, and FIG. 4 illustrates a system 319 that includes the delivery device 320 operably coupled to a hydraulic system 470 as well as a deployment assist 468. The details and operation of the hydraulic system 470 will be expanded upon herein with respect to FIGS. 9-13, and the details and operation of the deployment assist 468 will be expanded upon herein with respect to FIGS. 14-15. FIG. 5 is a cross-sectional view taken along line A-A of FIG. 3. FIG. 6 is an exploded view of the delivery device 320. The delivery device 320 includes an innermost shaft assembly 324, an inner steerable catheter 326 disposed over the innermost shaft assembly 324, and an outer steerable catheter 328 disposed over the inner steerable catheter 326. The innermost shaft assembly 324 incudes a capsule 322 for housing at least a portion of the prosthetic heart valve 100 during delivery thereof. The inner steerable catheter 326 includes a handle 327 at a proximal portion thereof for manipulation *in situ*, and the outer steerable catheter 328 includes a handle 329 at a proximal portion thereof for manipulation *in situ*. During delivery, the prosthetic heart valve 100 contained within the capsule 322 is steered by the inner steerable catheter 326 and the outer steerable catheter 328 into alignment within the native heart valve for which the prosthetic heart valve 100 serves as a replacement. The inner steerable catheter 326 may be controlled or steered independently from the outer steerable catheter 328 and provides the delivery device 320 with omnidirectional steering capabilities to direct the capsule 322.

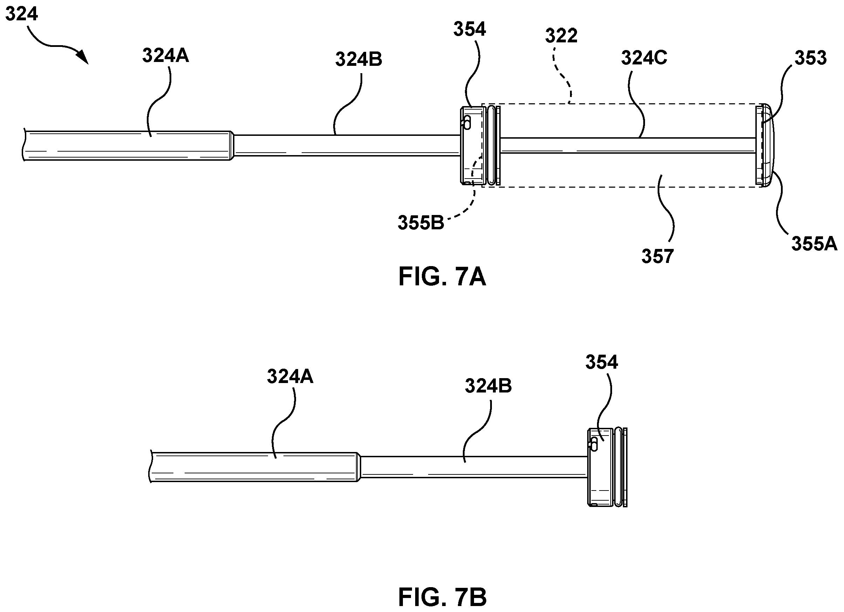

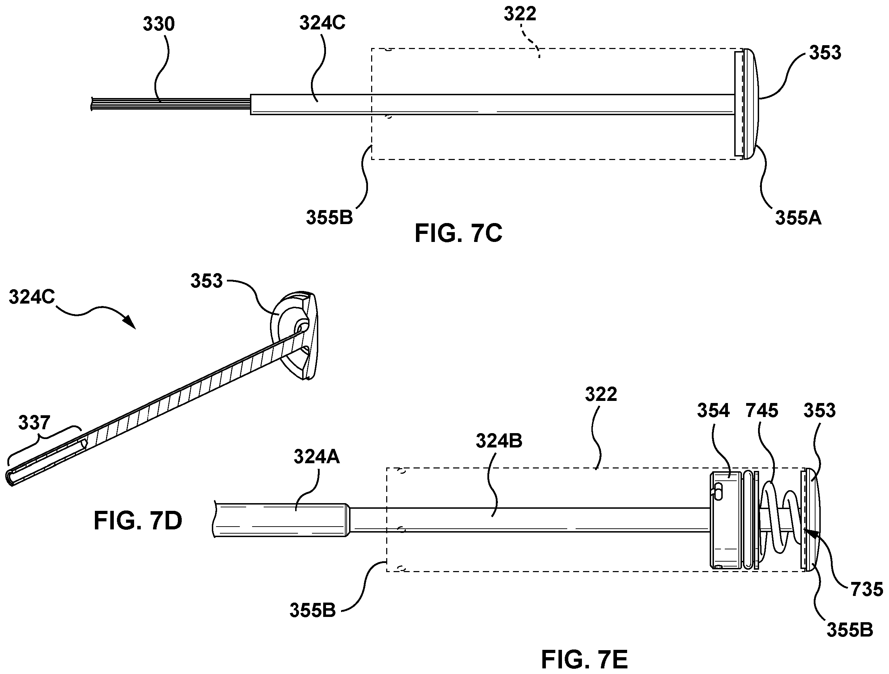

Components of the delivery device 320 will now be described in more detail. The innermost shaft assembly 324 includes a flexible shaft 324A, a piston mount 324B, a deployment piston 354, a tension cable 330, a distal shaft 324C, the capsule 322, and a capsule cap 353. At a proximal end thereof, as best shown in the exploded view of FIG. 6, the innermost shaft assembly 324 is fixedly secured to a manifold 325. FIG. 7A is an enlarged view of a distal portion of the innermost shaft assembly 324. The innermost shaft assembly 324 may be considered to include a first subassembly, shown in FIG. 7B and which includes the flexible shaft 324A, the piston mount 324B and the deployment piston 354, and a second subassembly, shown in FIG. 7C and which includes the tension cable 330, the distal shaft 324C, the capsule 322, and the capsule cap 353. The first and second subassemblies are coupled together in that the distal shaft 324C of the second subassembly slides or telescopes within the piston mount 324B of the first subassembly. In addition, the first and second subassemblies are coupled together via the manifold 325.

With respect to the first subassembly shown in FIG. 7B, the flexible shaft 324A is a flexible elongated tubular body that may include, for example, a flexible metal tetris or spring disposed within a polymer jacket. A distal end of the flexible shaft 324A is attached and fixed relative to a proximal end of the piston mount 324B, which is a rigid, tubular body that distally extends from the flexible shaft 324A. The deployment piston 354 is attached and fixed relative to the piston mount 324B. More particularly, the deployment piston 354 is disposed over and mounted to a distal end of the piston mount 324B.

With respect to the second subassembly shown in FIG. 7C, the capsule 322 is a tubular component having a closed or distal end 355A and an open or proximal end 355B. The capsule 322 may be rigid and made of metal. As will be described in more detail herein with respect to FIGS. 9-10, the capsule 322 is configured to house at least a portion of the prosthetic heart valve 100 during delivery. The distal end 355A of the capsule 322 is closed via the capsule cap 353. The capsule cap 353 may be integrally formed with the capsule 322 or may be a separate component attached thereto to form the closed distal end 355A. The distal shaft 324C is further attached to the capsule cap 353. FIG. 7D is a sectional view of the distal shaft 324C and the capsule cap 353. As shown in FIG. 7D, the distal shaft 324C may be integrally formed with the capsule cap 353, or in another embodiment, the distal end of the distal shaft 324C may be welded or otherwise attached to the capsule cap 353.

With additional reference to the cross-sectional view of FIG. 5, the tension cable 330 extends from the manifold 325 to the distal shaft 324C through the lumens of the flexible shaft 324A and the piston mount 324B. Reference number 331 is utilized in FIG. 5 to designate the lumen of the flexible shaft 324A. The lumens of the flexible shaft 324A and the piston mount 324B are in fluid communication with each other. A distal end of the tension cable 330 is secured or mounted within a proximal portion 337 (shown on FIG. 7D) of the distal shaft 324C. In an embodiment, the tension cable 330 is configured to be selectively tensioned (proximally or distally) by hydraulic force to enable translation of the capsule 322 either proximally or distally with respect to the piston mount 324B and the deployment piston 354 attached thereto. Depending on which hydraulic system is engaged (deployment or recapture), the tension cable 330 will translate under tension through the innermost shaft assembly 324, with the capsule 322 moving in a distal direction during deployment or moving in a proximal direction during recapture, as will be described in more detail herein.

The distal shaft 324C is received within the lumen of the piston mount 324B and may move or slide relative thereto in an axial or longitudinal direction. Stated another way, the distal shaft 324C telescopes within the piston mount 324B. The capsule 322 is concentrically disposed over the distal shaft 324C, and an annular chamber 357 (shown in FIG. 7A) is defined between an inner surface of the capsule 322, an outer surface of the distal shaft 324C, the deployment piston 354 and the capsule cap 353. As will be described in more detail with respect to FIGS. 9-11, the annular chamber 357 is a sealed cavity into which fluid can be introduced to increase fluid pressure therein and thereby move the capsule 322 away from the deployment piston 354, which remains stationary during fluid delivery as described below.

In any embodiment hereof, and as shown in FIG. 7E, the inner steerable catheter 326 may further include a distal push assist 735 disposed at the distal end of the capsule 322. The distal push assist 735 includes a spring 745 having a first end positioned at and attached to the capsule cap 353 and a second end positioned at and secured to the deployment piston 354. The distal push assist 735 is configured to assist the hydraulic deployment system 470 by lowering the required hydraulic pressure to move the capsule 322 away from the deployment piston 354 and to deploy the heart valve prosthesis 100. During loading of the heart valve prosthesis 100, the spring 745 is compressed via the recapture hydraulic circuit (which is described in more detail below) imparting stored energy into the compressing of the spring 745. During deployment, the energy from the compressed spring 745 is released and assists in moving the capsule 322 relative to the deployment piston 354, thereby reducing the necessary deployment pressure. In an embodiment, the distal push assist 735 is configured to reduce the necessary deployment pressure by 10atm or 150psi.

The inner steerable catheter 326 is disposed over the innermost shaft assembly 324 such that an annular lumen 332 (shown on FIG. 5) is defined between an outer surface of the innermost shaft assembly 324 and an inner surface of the inner steerable catheter 326 along an entire length of the inner steerable catheter 326. The innermost shaft assembly 324 is slidingly disposed within the inner steerable catheter 326 such that relative axial movement is permitted therebetween as will be described in more detail below. As used herein, "slidably" generally denotes back and forth movement in a longitudinal direction along or generally parallel to a central longitudinal axis L_{A} of the delivery device 320. The inner steerable catheter 326 includes a flexible, steerable tubular component or shaft 334, the handle 327 fixedly secured to a proximal end 336 of the shaft 334, an inner distal flex component 340 extending distally from a distal end 338 of the shaft 334, and a first pullwire 342. The shaft 334 can assume various forms conventionally employed, and in some embodiments can be a braided catheter surrounded by a polymer outer layer or jacket. The inner distal flex component 340 is secured to and extends distally from the shaft 334 and can be configured to exhibit flexibility and/or hoop strength characteristics differing from that of the shaft 334. In an embodiment, the inner distal flex component 340 is a metal tube with a laser cut pattern that facilitates flexing or bending of the inner distal flex component 340. A cap 341 (shown on FIG. 3) is attached to a distal end of the inner distal flex component 340. The cap 341 is an annular component that permits the innermost shaft assembly 324 to slide therethrough. The handle 327 includes an actuator 327A that is accessible to the user and may be manipulated to control flexing or bending of the inner distal flex component 340 of the shaft 334. More particularly, as will be explained in more detail herein, the first pullwire 342 is attached to and extends between the handle 327 and the cap 341 attached to the inner distal flex component 340. The first pullwire 342 is selectively tensioned by the user to bend the inner distal flex component 340. The inner steerable catheter 326 is configured to transition between a non-flexed configuration when the first pullwire 342 is not tensioned and a flexed configuration in which the first pullwire 342 is tensioned.

The handle 327 includes the actuator 327A for tensioning the first pullwire 342. The handle 327 can have any shape or size appropriate for convenient handling by a user. The actuator 327A is coupled to the proximal end of the first pullwire 342 and is constructed to provide selective proximal retraction and distal advancement of the first pullwire 342. Stated another way, the actuator 327A is coupled to the proximal end of the first pullwire 342 and is constructed to selectively push or pull the first pullwire 342. The actuator 327A may assume any construction that is capable of providing the desired pullwire actuation functionality. In an embodiment, the actuator 327A is configured as a rotatable knob that is rotated in a first direction (i.e., clockwise) to proximally retract the first pullwire 342 and apply tension thereto, and is rotated in a second, opposing direction (i.e., counter-clockwise) to distally advance the first pullwire 342 and remove or release tension therefrom, such as the rotatable knob described in U.S. Patent No. 10,188,833 to Bolduc et al., filed December 8, 2015, or the rotatable knob described in U.S. Patent No. 6,607,496 to Poor et al., filed on September 12, 2000, each of which is assigned to the same assignee as the present disclosure . In another embodiment, the actuator 327A may be configured as a button such as those described in U.S. Patent No. 10,278,852 to Griffin, filed on March 10, 2016, which is assigned to the same assignee as the present disclosure.

The outer steerable catheter 328 is slidably disposed over the inner steerable catheter 326 such that an annular lumen 343 (shown on FIG. 5) is defined between an outer surface of the inner steerable catheter 326 and an inner surface of the outer steerable catheter 328 along an entire length of the outer steerable catheter 328. The outer steerable catheter 328 includes a flexible, steerable tubular component or shaft 344, the handle 329 fixedly secured relative to a proximal end 346 of the shaft 344, an outer distal flex component 350 extending distally from a distal end 348 of the shaft 344, and a second pullwire 352. The shaft 344 can assume various forms conventionally employed, and in some embodiments can be a braided catheter surrounded by a polymer outer layer or jacket. The outer distal flex component 350 is secured to and extends distally from the shaft 344 and can be configured to exhibit flexibility and/or hoop strength characteristics differing from that of the shaft 344. In an embodiment, the outer distal flex component 350 is formed from a metal tube with a laser cut pattern that facilitates flexing or bending of the outer distal flex component 350. A cap 351 is attached to a distal end of the outer distal flex component 350. The cap 351 is an annular component that permits the inner steerable catheter 326 to slide therethrough. The handle 329 includes an actuator 329A that is accessible to the user and may be manipulated to control steering of the outer distal flex component 350 of the shaft 344. More particularly, as will be explained in more detail herein, the second pullwire 352 is attached to and extends between the handle 329 and the cap 351 of the outer distal flex component 350. The second pullwire 352 is selectively tensioned by the user to bend the outer distal flex component 350. The outer steerable catheter 328 is configured to transition between a non-flexed configuration when the second pullwire 352 is not tensioned and a flexed configuration in which the second pullwire 352 is tensioned.

The handle 329 includes the actuator 329A for tensioning the second pullwire 352. The handle 329 can have any shape or size appropriate for convenient handling by a user. The actuator 329A is coupled to the proximal end of the second pullwire 352 and is constructed to provide selective proximal retraction and distal advancement of the second pullwire 352. Stated another way, the actuator 329A is coupled to the proximal end of the second pullwire 352 and is constructed to selectively push or pull the second pullwire 352. The actuator 329A may assume any construction that is capable of providing the desired pullwire actuation functionality. In an embodiment, the actuator 329A is configured as a rotatable knob that is rotated in a first direction (i.e., clockwise) to proximally retract the second pullwire 352 and apply tension thereto, and is rotated in a second, opposing direction (i.e., counter-clockwise) to distally advance the second pullwire 352 and remove or release tension therefrom, such as the rotatable knob described in U.S. Patent No. 10,188,833 to Bolduc et al., filed December 8, 2015, or the rotatable knob described in U.S. Patent No. 6,607,496 to Poor et al., filed on September 12, 2000, each of which is assigned to the same assignee as the present disclosure. **In** another embodiment, the actuator 329A may be configured as a button such as those described in U.S. Patent No. 10,278,852 to Griffin, filed on March 10, 2016, which is assigned to the same assignee as the present disclosure.

As stated above, the delivery device 320 is operatively coupled to the hydraulic system 470 (see FIG. 4). The hydraulic system 470 is utilized to axially or longitudinally move the capsule 322 relative to the prosthetic heart valve 100. More particularly, the hydraulic system 470 includes a deployment pressure delivery device 472 and a recapture pressure delivery device 473. The deployment pressure delivery device 472 is configured to deliver hydraulic fluid to the annular chamber 357 of the delivery device 320 in order to drive the capsule 322 in a distal direction, thereby deploying the prosthetic heart valve 100, as will be described in more detail herein. The deployment pressure delivery device 472 is configured to be fluidly coupled to a deployment valve 463 of the manifold 325. The recapture pressure delivery device 473 is configured to deliver hydraulic fluid to a recapture chamber 461 of the delivery device 320 in order to drive the capsule 322 in a proximal direction, thereby recapturing the prosthetic heart valve 100, as will be described in more detail herein. The recapture pressure delivery device 473 is configured to be fluidly coupled to a recapture valve 464 of the manifold 325. In an embodiment, the deployment pressure delivery device 472 and the recapture pressure delivery device 473 are inversely related to each other. For example, when the delivery device 320 is in the delivery configuration, the deployment pressure delivery device 472 is full (as the annular chamber 357 is empty), while the recapture pressure delivery device 473 is empty (as the recapture chamber 461 is full). Conversely, when the delivery device 320 is in the deployed configuration, the deployment pressure delivery device 472 is empty (as the annular chamber 357 is full), while the recapture pressure delivery device 473 is full (as the recapture chamber 461 is empty). The fluid paths of the recapture chamber 461 and the annular chamber 357 are independent of one another. However, it is envisioned that in some embodiments, the recapture chamber 461 and the annular chamber 357 are fluidly connected to one another, allowing for the recapture chamber 461 and the annular chamber 357 to change volume inversely based on a single source of hydraulic fluid. In an embodiment, each of the pressure delivery devices 472, 473 may be a pump or a syringe-type inflator. In another embodiment, the pressure delivery devices 472, 473 may be integrated into an inflation device that is configured to be coupled to a flow reverser that selects which hydraulic cylinder or chamber 461, 357 to pressurize, while venting the opposing or non-selected cylinder or chamber 461, 357.

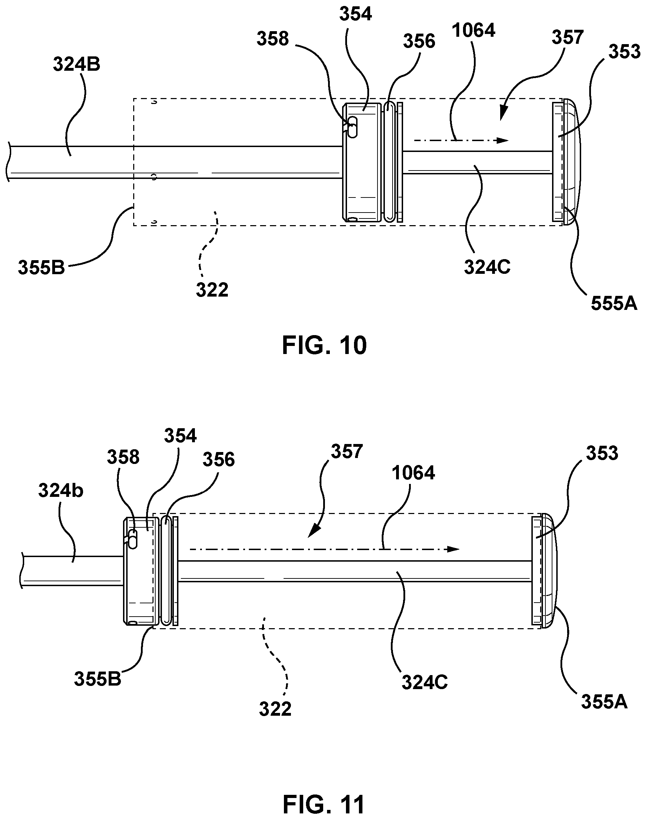

Turning now to FIGS. 8-11, the operation of the hydraulic system 470 and the capsule 322 for deploying an implant such as prosthetic heart valve 100 will be described in more detail. FIG. 8 is a perspective view of the deployment piston 354, and FIG. 9 illustrates a delivery configuration of the prosthetic heart valve 100 after being loaded onto the deployment piston 354 and disposed within the capsule 322 for delivery thereof. FIGS. 10 and 11 illustrate movement of the capsule 322 relative to the deployment piston 354 during deployment of the prosthetic heart valve 100. The prosthetic heart valve 100 is depicted in FIG. 9, but is not shown in FIGS. 10 and 11 for sake of clarity, as these figures are primarily provided to illustrate the relative movement of the capsule 322 relative to the deployment piston 354.

The deployment piston 354 is shown removed from the delivery device 320 in FIG. 8. The deployment piston 354 is an annular component that defines an opening or central bore 333 such that the deployment piston 354 is configured to be disposed over and attached to a distal end of the piston mount 324B. The deployment piston 354 includes a plurality of slots or recesses 358 configured to receive the attachment bars 112 of the prosthetic heart valve 100. The deployment piston 354 also includes an annular groove 360 on an outer surface thereof. A seal 356 (shown in FIGS. 9-11) is disposed within the annular groove 360. The seal 356 is thus coupled to the deployment piston 354 and functions to provide a fluid seal between the deployment piston 354 and an inner surface of the capsule 322. The seal 356 may be, for example, an O-ring. When fluid is present in the annular chamber 357, the seal 356 prevents fluid from leaking out between the outer surface of the deployment piston 354 and the inner surface of the capsule 322.

Referring now to FIG. 9, the prosthetic heart valve 100 is loaded onto the deployment piston 354 and disposed within the capsule 322 for delivery thereof. It will be understood by one of ordinary skill in the art that other delivery configurations of the prosthetic heart valve 100 are contemplated and the illustrated configuration is only exemplary. For example, the capsule 322 is not required to extend over the full length of the prosthetic heart valve 100. In another embodiment (not shown), a portion of the prosthetic heart valve 100 may extend proximally from the open or proximal end 355b of the capsule 322 and may be radially compressed for delivery within the vasculature via a suture. In the delivery configuration depicted in FIG. 9, the capsule 322 is shown in a first position relative to the deployment piston 354 in which the deployment piston 354 abuts against or is disposed directly adjacent to the capsule cap 353. In this first position, the capsule 322 is positioned so as to compressively retain at least a portion of the prosthetic heart valve 100. The prosthetic heart valve 100 is coupled to the deployment piston 354 via the attachment bars 112 being disposed within the plurality of slots 358. In the depicted embodiment, the capsule 322 has a length that is greater than or substantially equal to a length of the prosthetic heart valve 100 such that the full length of the prosthetic heart valve 100 is radially compressed by the capsule 322. However, in another embodiment (not shown), the capsule 322 may have a length that is shorter than a length of the prosthetic heart valve 100 such that a proximal portion of the prosthetic heart valve 100 extends proximally out of the proximal end 355b of the capsule 322. In FIG. 9, the distal shaft 324C is concealed from view since the piston mount 324B extends thereover. In this delivery configuration of FIG. 9, the clinician may then insert the distal end of the delivery device 320 into the patient and navigate the capsule 322 through the vasculature of the patient to the desired location within the patient's heart.

Once in the proper position, the clinician may then use the deployment pressure delivery device 472 to begin to fill the annular chamber 357, moving capsule 322 axially relative to the deployment piston 354. More particularly, with reference to FIGS. 10 and 11, the capsule 322 is configured to be distally advanced relative to the deployment piston 354 in order to incrementally release and deploy the prosthetic heart valve 100 from the capsule 322. Via the manifold 325, fluid is injected from the deployment pressure delivery device 472 into the innermost shaft assembly 324 in order to drive the capsule 322 distally. The prosthetic heart valve 100 remains in a stationary longitudinal position relative to the native valve while the capsule 322 is driven distally, thereby increasing the precision of deployment.

More particularly, the manifold 325 is connected to the deployment pressure delivery device 472 of the hydraulic system 470 via the deployment valve 463. The deployment pressure delivery device 472 is fluidly connected to the annular chamber 357 within the capsule 322 via the lumens of the flexible shaft 324A and the piston mount 324B (which are in fluid communication with each other). Fluid enters the annular chamber 357 via the outlet of the piston mount 324B, around the distal shaft 324C through the annular space or lumen defined between the outer surface of the distal shaft 324C and the inner surface of the piston mount 324B. As shown by the directional arrow 1064 in FIG. 10, as the annular chamber 357 fills with fluid, the capsule 322 is distally advanced with respect to the deployment piston 354. FIG. 10 illustrates the capsule 322 at a second position relative to the deployment piston 354, in which the deployment piston 354 is disposed within the capsule 322 at approximately a midportion thereof. The annular chamber 357 between the deployment piston 354 and the capsule cap 353 is filled with fluid from the deployment pressure delivery device 472. The deployment piston 354, which is attached and fixed to the piston mount 324B and the flexible shaft 324A, remains stationary as the capsule 322 and distal shaft 324C move in an axial direction. The deployment piston 354 (and piston mount 324B and flexible shaft 324A) may be held in place by holding the manifold 325 stationary during fluid delivery.

The fluid continues to fill the annular chamber 357 until the capsule 322 reaches a third position relative to the deployment piston 354 depicted in FIG. 11, in which the deployment piston 354 is partially disposed within the capsule 322 and is directly adjacent to the proximal end 355b of the capsule 322. The annular chamber 357 between the deployment piston 354 and the capsule cap 353 is filled with fluid from the deployment pressure delivery device 472. At this third position, the plurality of slots 358 of the deployment piston 354 are no longer covered by the capsule 322. With the plurality of slots 358 exposed, the attachment bars 112 of the prosthetic heart valve 100 are permitted to decouple from the deployment piston 354. Accordingly, via movement of the capsule 322, the prosthetic heart valve 100 is unsheathed from the capsule 322. When the capsule 322 no longer covers or extends over the attachment bars 112, the attachment bars 112 are free or permitted to pop out of the slots 358 of the deployment piston 354 to decouple the prosthetic heart valve 100 from the deployment piston 354. Thus, once the prosthetic heart valve 100 is no longer covered by the capsule 322, the prosthetic heart valve 100 is permitted to radially self-expand towards the expanded configuration of FIG. 1.

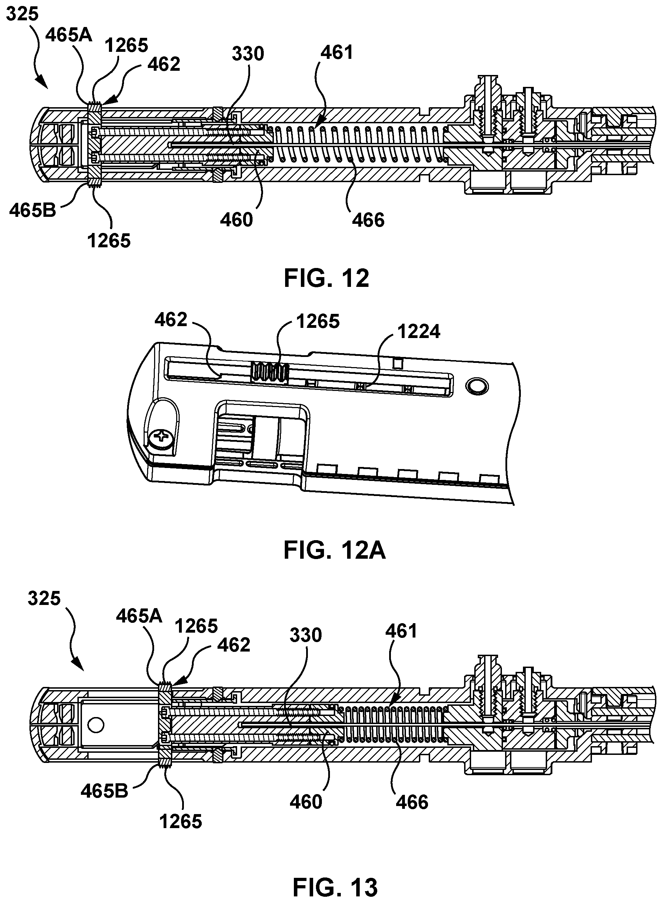

Turning now to FIGS. 12-13, additional features of the hydraulic system 470 during deployment of an implant such as the prosthetic heart valve 100 will be described. The manifold 325 includes a recapture piston 460, the recapture chamber 461, and a hard stop 462. The recapture chamber 461 is disposed within the manifold 325 and is configured to house the recapture piston 460 therein. The recapture chamber 461 is fluidly coupled to the recapture valve 464 and is configured to fill with hydraulic fluid from the recapture pressure delivery device 473. As the hydraulic fluid from the recapture pressure delivery device 473 flows into and/or exits from the recapture chamber 461, the recapture piston 460 is configured to move axially within the recapture chamber 461.

The recapture piston 460 is attached to the hard stop 462. The hard stop 462 includes a pair of opposing radial protrusions 465A, 465B which extend from the recapture piston 460 and are slidingly disposed within a pair of opposing hard stop channels 1224. One of the hard stop channels 1224 is shown on the enlarged perspective view of FIG. 12A, and it will be understood by one of ordinary skill in the art that another hard stop channel 1224 is formed at an opposing location to the one shown on FIG. 12A. The radial protrusions 465A, 465B of the hard stop 462 move axially within the hard stop channels 1224 as the recapture piston 460 moves axially. The radial protrusions 465A, 465B of the hard stop 462 are accessible to the clinician on the outer surface of the manifold 325, allowing for the clinician to both control the position of the hard stop 462 within the manifold 325 and to monitor the transition of the delivery device 320 from the delivery configuration to the deployed configuration. As the radial protrusions 465A, 465B of the hard stop 462 move within the hard stop channels 1224, the length of the hard stop channels 1224 restrict the hard stop 462 and the recapture piston 460 attached thereto from moving axially past a certain point, ensuring that the delivery device 320 does not overextend past the deployment configuration. As best shown on the enlarged perspective view of FIG. 12A, the radial protrusions 465A, 465B of the hard stop 462 each include a series of threads 1265 positioned on the outer surface thereof as to allow for the threaded deployment assist 468 to be used to control the movement of the hard stop 462, as will be described in more detail below with respect to FIGS. 14-15.

FIG. 12 is a sectional view of the manifold 325 when the delivery device 320 is in a delivery configuration. While in the delivery configuration, the recapture piston 460 is disposed at a proximal end of the recapture chamber 460 to allow for the recapture chamber 461 to contain the most amount of hydraulic fluid. Further, the hard stop 462 is positioned at the proximal end of the hard stop channel 1224 and is attached to the recapture piston 460 such that the hard stop 462 will move axially as the recapture piston 460 moves axially.

FIG. 13 is a sectional view of the manifold 325 when the delivery device 320 is in the deployed configuration. In the deployed configuration, the recapture piston 460 is disposed at a distal end of the recapture chamber 461 and the recapture chamber 461 contains a reduced volume of hydraulic fluid compared to the initial volume thereof, and the annular chamber 357 contains an increased volume of hydraulic fluid compared to the initial volume thereof. The hard stop 462 is positioned at the distal end of the hard stop channel 1224, restricting the ability of the hard stop 462 and the recapture piston 460 from moving further distally. By restricting further distal movement of the recapture piston 460, the hard stop 462 prevents the capsule 322 from moving further distally and potentially disengaging from the deployment piston 354.

The recapture piston 460 is operably coupled to the capsule 322 via the tension cable 330, meaning that movement of either will place an axial force onto the opposing component. More particularly, a proximal end of the tension cable 330 is attached to the recapture piston 460 and a distal end of the tension cable 330 is attached the distal shaft 324C, which is, subsequently coupled to the capsule cap 353 as described above. The tension cable 330 remains taut or under tension between the recapture piston 460 and the distal shaft 324C, resulting in a force that encourages the recapture piston 460 and distal shaft 324C to move together. Further, in some embodiments, the movement of the recapture piston 460 may be supplemented by a spring 466. The spring 466 pushes against the recapture piston 460 and biases the recapture piston 460 towards a proximal end of the recapture chamber 461. The recapture piston 460 and the spring 466 provide an opposing or dampening force during deployment of the prosthetic heart valve 100. Prior to deployment of the prosthetic heart valve 100, the recapture chamber 461 is filled with hydraulic fluid, and this hydraulic fluid must be pushed out of the recapture chamber 461 as the recapture piston 460 is pulled distally during deployment of the prosthetic heart valve 100. More particularly, as the annular chamber 357 fills with hydraulic fluid during deployment of the prosthetic heart valve 100 and the capsule 322 is driven distally, the recapture piston 460 is also pulled distally since the proximal end of the tension cable is attached thereto. However, although recapture piston 460 is being pulled distally, the hydraulic fluid within the recapture chamber 461 and the spring 466 provide an opposing force on the recapture piston 460 and the tension cable 330 and thereby dampen the deployment forces during movement of the deployment piston 354. This dampening effect is desirable because the hydraulic system may be prone to axial jumping due to uneven or unpredictable changes in the hydraulic fluid pressure. To explain further, forces such as friction, uneven fluid pressure, and the like may cause the capsule 322 to "jump" when being driven distally during deployment of the prosthetic heart valve 100. In other words, a "jump" in the system may occur when the pressure of the hydraulic fluid provided to the annular chamber 357 exceeds the forces required to move the annular chamber 357 distally, causing the capsule 322 to move quickly, which may rapidly reduce the pressure of the hydraulic fluid until it can no longer overcome the force required to move the capsule 322. Subsequently, in order to move the capsule 322, the clinician will be required to start increasing the pressure of the hydraulic fluid again. In such a scenario, the amount the capsule 322 moves relative to the deployment piston 354 may be unpredictable, increasing the risk of unintentional full deployment of the prosthetic heart valve 100 and an overall reduction in control of the deployment process. Therefore, by dampening the movement of the capsule 322 via the hydraulic fluid within the recapture chamber 461 and the spring 466, a more predictable fluid pressure is maintained within the system 319.

As stated above, the system 319 further includes the deployment assist 468, which is shown in section on FIG. 4. The deployment assist 468 is best shown in the perspective views of FIGS. 14 and 15. The deployment assist 468 is disposed at the proximal end of the manifold 325 and is configured to apply an axial force to the recapture piston 460 in order to help transition the delivery device 320 from the delivery configuration of FIG. 12 to the deployed configuration of FIG. 13. In some situations, the force required to start the transition process will be greater than the force that the hydraulic system 470 is able to place onto the capsule 322. The increased initiation force may be due to a variety of factors, such as static friction, dynamic friction, external forces, and the like. Therefore, as increasingly smaller delivery device and prosthetic heart valve dimensions are developed, the more difficult it may be to create enough hydraulic pressure to drive the capsule 322 distally to begin the transition. The deployment assist 468 is disposed over and coupled to the hard stop 462, and functions to apply a mechanical force to the hard stop 462 and the recapture piston 460 attached thereto to start or begin driving the capsule 322 distally. In some embodiments, the deployment assist 468 along is utilized to start or begin driving the capsule 322 distally. Stated another way, the deployment assist 468 may be the sole means for at least a portion of the deployment of the capsule 322. In another embodiment, the deployment assist 468 will be used in conjunction with the hydraulic system to start or begin driving the capsule 322 distally.

The deployment assist 468 includes an endcap 1461 and a knob 1467. The endcap 1461 is disposed over an outer surface of the manifold 325 and is configured to remain rotationally and axially fixed relative to the housing of the manifold 325. More particularly, a locking aperture 1463 is formed on the endcap 1461 and is configured to receive a pin 1464 therethrough. Stated another way, the pin 1464 may be inserted through the aperture 1463 and into the manifold 325. The pin 1464 functions secure the endcap 1461 to the manifold 325 and restricts or prevents the endcap 1461 from moving in an axial or longitudinal direction or rotating relative to the manifold 325.

The knob 1467 is attached to the endcap 1461. The knob 1467 is configured to rotate relative to both the manifold 325 and the endcap 1461, but the knob 1467 is longitudinally fixed relative to both the manifold 325 and the endcap 1461. The knob 1467 includes a threaded inner surface or a series of threads 1564 on an inner surface thereof that is configured to interact with the threads 1265 of the hard stop 462. The threads 1564 of the knob 1467 are configured to interact or mate with the threads 1265 of the hard stop 462. Since the longitudinal or axial position of the knob 1467 is fixed due to the endcap 1461 being disposed over the proximal end of the manifold 325, rotation of the knob 1467 results in longitudinal or axial movement of the hard stop 462 due to the interaction between the threads 1564 and the threads 1265. Thus, the knob 1467 is configured to allow a clinician to selectively control the axial movement of the hard stop 462 by rotating the knob 1467.

It is envisioned, that in some embodiments, the system 319 will use the deployment assist 468 to at least begin the process of deploying the prosthetic heart valve 100. In some embodiments, the highest force of resistance is at the beginning of the deployment process, and, therefore, it is beneficial for the deployment assist 468 to be used at the initial stages of deployment. To deploy the heart valve prosthesis once the capsule 322 is positioned at the target location, the clinician may begin to increase the amount of hydraulic fluid within the annular chamber 357 by depressing the deployment pressure delivery device 472 of the hydraulic system 470. In the event that the device will not begin to transition due to aforementioned forces in the capsule 322, the clinician may rotate the knob 1467 of the deployment assist 468 in order to mechanically force the hard stop 462 distally within the hard stop channel 1224, and subsequently, will apply an axial force onto the tension cable 330 which will force the capsule 322 to move distally and to begin the transition process. Stated another way, the tension cable 330 will transmit an additional axial force to the capsule 322 to supplement the fluid pressure from the hydraulic fluid, and the combination of mechanical and hydraulic forces are sufficient to initiate movement of the capsule 322 distally.

In the event of an emergency, such as an unforeseen complication, there may be a need for the clinician to quickly recapture the prosthetic heart valve 100 and/or remove the delivery device 320 from the patient. Therefore, the deployment assist 468 is configured to be removable from the manifold 325 in order to allow direct access to the hard stop 462 if needed. The pin 1464 is removable from the locking aperture 163. After the pin 1464 is removed, the deployment assist 468 (including both the endcap 1461 and the knob 1467) can slide off the proximal end of the manifold 325 to expose the hard stop 462. The clinician may then remove both the endcap 1461 and the knob 1467, allowing for the hard stop 462 to be manually or hydraulically returned to the delivery configuration for repositioning of the prosthetic heart valve 100 and/or to allow for the delivery device 320 to be removed from the patient's body.

In addition to providing a mechanical assist for deployment or movement of the capsule 322, the deployment assist 468 may also limit unexpected or unintentional movement of the capsule 322. For example, forces such as friction, uneven pressure, and the like may cause the capsule 322 to "jump" during transition. As described above, a "jump" in the system 319 may occur when the pressure of the hydraulic fluid provided to the annular chamber 357 exceeds the forces required to move the capsule 322 distally, causing the capsule 322 to move quickly. When the capsule moves too quickly, the pressure of the hydraulic fluid within the annular chamber 357 is rapidly reduced until it can no longer overcome the force required to move the capsule 322, requiring the clinician to then start increasing the pressure of the hydraulic fluid again. The deployment assist 468 may be further used to reduce the risk of the delivery device 320 "jumping" during transition by using the deployment assist 468 to control the rate of axial movement of the capsule 322. More particularly, the knob 1467 may be rotated at a consistent rate with hydraulic fluid is delivered to the annular chamber 357 at the same rate. The hydraulic pressure within the annular chamber 357 thus increases at the same rate as the capsule 322 is driven distally, which allows the hydraulic system 470 to maintain a more uniform hydraulic pressure profile through the process of the transition without a rapid and unexpected change in volume.

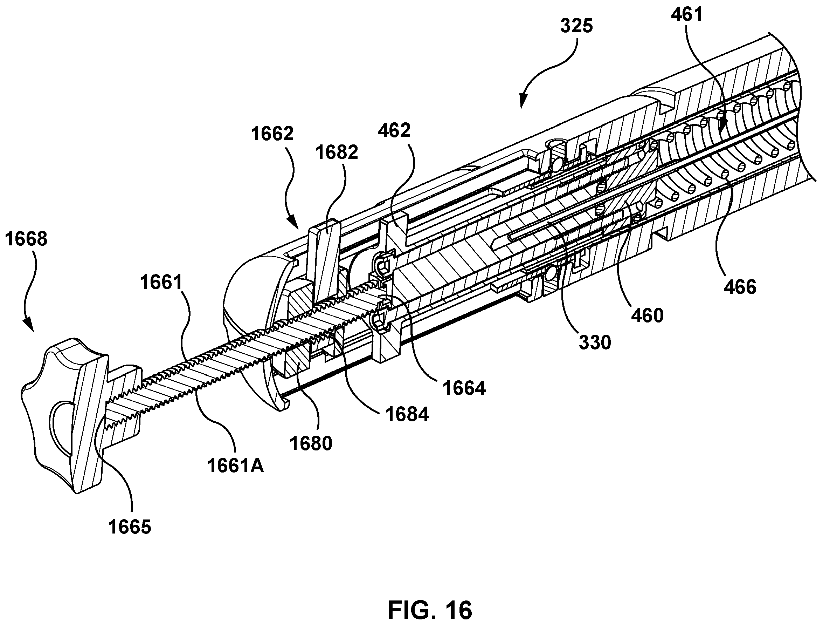

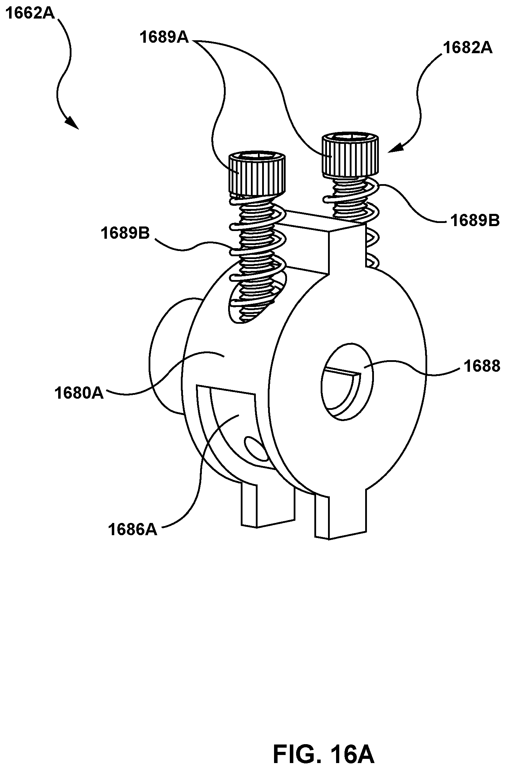

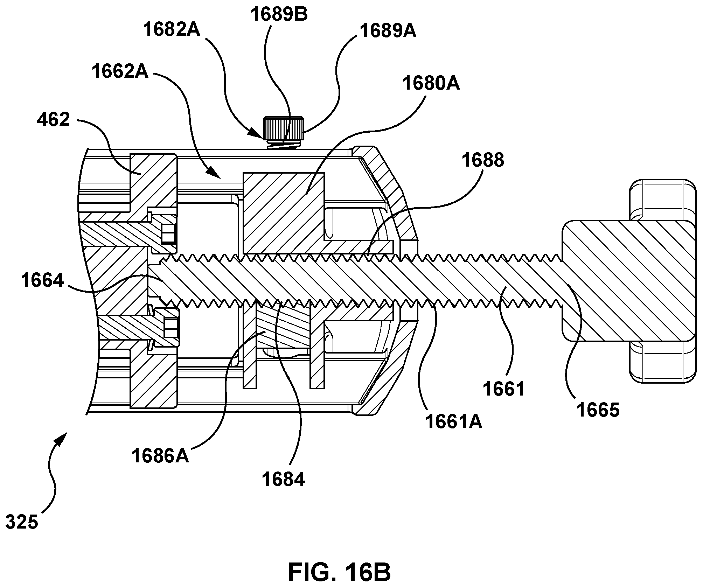

Although the deployment assist 468 includes the knob 1467 for driving the hard stop 462 distally, other embodiments of deployment assist components are contemplated to be used in conjunction with the hydraulic system to start or begin driving the capsule 322 distally. Another embodiment of a deployment assist 1668 integrated into the manifold 325 of the delivery device 320 is shown in the sectional view of FIG. 16. The deployment assist 1668 includes a threaded shaft 1661 and a release mechanism 1662. The threaded shaft 1661 includes a plurality of threads 1661A on an outer surface thereof, and the release mechanism 1662 includes a plurality of threads 1684 on an inner surface thereof. The threads 1661A of the threaded shaft 1661 mate or engage with the threads 1684 of the release mechanism 1662. A distal end 1664 of the threaded shaft 1661 is coupled to the recapture piston 460, and the threaded shaft 1661 extends proximally through and out of the manifold 325, allowing for a clinician to interact with a proximal end 1665 of the threaded shaft 1661. The distal end 1664 of the threaded shaft 1661 is coupled to the recapture piston 460 such that the threaded shaft 1661 is permitted to rotate relative to the recapture piston 460, and so that the threaded shaft 1661 and the recapture piston 460 move together as an assembly when translated in an axial or longitudinal direction.

To explain in detail, the release mechanism 1662 is disposed at least partially within the manifold 325, adjacent to the proximal end thereof, and extends substantially perpendicular to the threaded shaft 1661. Further, the release mechanism 1662 generally includes a body 1680 and an engagement tab 1682. The body 1680 is positioned within the proximal end of the manifold 325 and is attached or secured to an inner surface of the manifold 325 such that the body 1680 is fixed relative to the manifold 325. The engagement tab 1682 includes the threads 1684 of the release mechanism 1662 as discussed above and is disposed at least partially within the manifold 325 and extends substantially perpendicular to the threaded shaft 1661. The engagement tab 1682 is disposed within the manifold 325 so as to be movable relative thereto, as will be described in more detail below. A portion of the engagement tab 1682 extends out of the manifold 325 so that the engagement tab 1682 can be manipulated and moved by the user.

More particularly, the engagement tab 1682 can be selectively engaged and disengaged by the clinician. In a first position, the threads 1684 positioned on the engagement tab 1682 are not engaged with the threads 1661A of the threaded shaft 1661. While in a second position, the threads 1684 on the engagement tab 1682 are engaged with the threads 1661A of the threaded shaft 1661. When the engagement tab 1682 is in the second position and the threads 1684 are engaged with the threads 1661A of the threaded shaft 1661, the mating threaded relationship between the engagement tab 1682 and the threaded shaft 1661 causes axial or longitudinal translation of the threaded shaft 1661 when the threaded shaft is rotated, while the engagement tab 1682 remains stationary in the second position. When the threaded shaft 1661 is advanced in a longitudinal direction, the recapture piston 460 coupled thereto is concurrently moved therewith. Thus, the clinician can rotate the threaded shaft 1661 as to move the recapture piston 460 axially, which also drives the capsule 322 axially as described above in order to assist the hydraulic system 470 in moving the capsule 322. In another embodiment (not shown), only a distal portion of the threaded shaft 1661 is threaded as to use the deployment assist 1668 only during the initial deployment and using solely the hydraulic system to complete the deployment of the prosthetic heart valve 100.

In the event of an emergency, such as an unforeseen complication, there may be a need for the clinician to quickly recapture the prosthetic heart valve 100 and/or remove the delivery device 320 from the patient. Therefore, the engagement tab 1682 may be disengaged from the threaded shaft 1661A such that the threads 1684 on the engagement tab 1682 are spaced from and no longer engaged with the threads 1661A of the threaded shaft 1661 and thereby allowing for the threaded shaft 1661 to freely move or translate in the axial or longitudinal direction. Particularly, the clinician may then axially or longitudinally manually translate the threaded shaft 1661 and recapture piston 460 proximally, which, subsequently, moves the capsule 322 proximally to recapture the prosthetic heart valve 100 if desired.

An exemplary embodiment of the release mechanism 1662 is shown in FIGS. 16A and 16B. In FIG. 16A, a release mechanism 1662A is shown in a perspective view and removed from the manifold 325, and FIG. 16B shows the release mechanism 1662A disposed within the manifold 325. In this embodiment, the release mechanism 1662A includes a body 1680A, an engagement tab 1682A, and a threaded portion 1686A. The body 1680A is secured or attached to an inner surface of the manifold 325 so that the body 1680 does not move relative to the manifold 325. The body 1680A further includes a lumen 1688 configured to receive the threaded shaft 1661. The threaded portion 1686A is positioned or disposed within the body 1680 and includes threads 1684A of the release mechanism 1662A that mate or engage with the threads 1661A of the threaded shaft 1661.

A portion of the engagement tab 1682A is positioned within the body 1680 and extends outward from the body 1680A perpendicularly to the lumen 1688 of the body 1680A. The engagement tab 1682A is attached to the threaded portion 1686A so that the threaded portion 1686A moves with the engagement tab 1682A. In a first or engaged position of the release mechanism 1662A, the threaded portion 1686A mates with the threaded shaft 1661, allowing for the clinician to advance or move the hard stop 462, the recapture piston 460, and the capsule 322 axially by rotating the threaded shaft 1661 as described above with respect to FIG. 16. When the release mechanism 1662A is in the first position, the threaded shaft 1661 may only move distally by rotating the threaded shaft 1661. While in a second or non-engaged position of the release mechanism 1662A, the threaded portion 1686A is separated or radially spaced apart from the threaded shaft 1661 such that rotation of the threaded shaft 1661 by a clinician no longer results in axial movement of the hard stop 462.

In the depicted embodiment of the engagement tab 1682A, the engagement tab 1682A includes two posts 1689A, with each post 1689A including a spring 1689B concentrically surrounding the post 1689A and being attached thereto. The springs 1689B are configured in an extended configuration to bias the engagement tab 1682A in the first or engaged position of the release mechanism 1662A. In order to transition the release mechanism 1662A to the second or non-engaged position, the clinician may depress the engagement tab 1682A. When the engagement tab 1682A is depressed, the posts 1689A are depressed and the springs 1689B compress. In turn, when the engagement tab 1682A is depressed, the engagement tab 1682A pushes or displaces the threaded portion 1686A such that the threaded portion 1686A disengages from the threaded shaft 1661. When the threaded portion 1686A is disengaged from the threaded shaft 1661, the threaded shaft 1661 may move axially without being rotated as explained above with respect to FIG. 16.

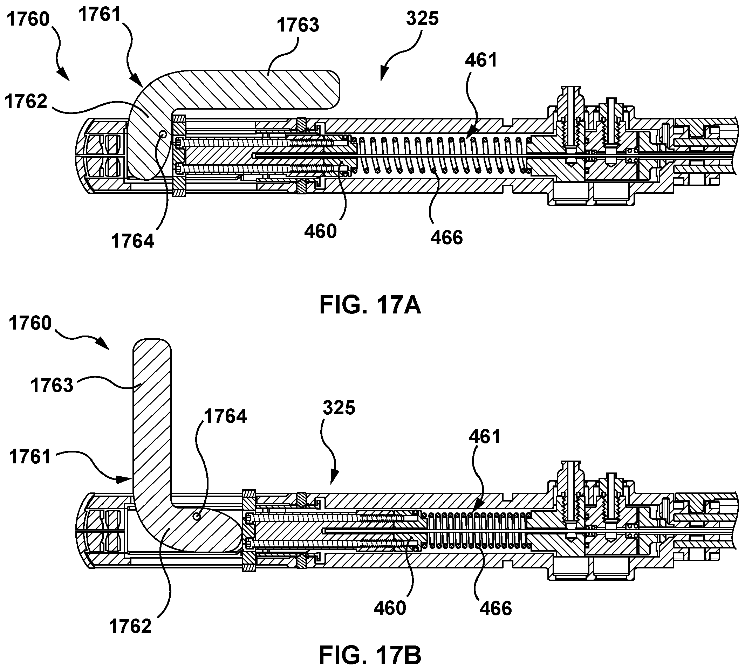

FIGS. 17A and 17B shows another embodiment of a deployment assist 1760 integrated into the manifold 325 of the delivery device 320. The deployment assist 1760 is configured to place an additional, mechanical force on the capsule 322 to assist the hydraulic system 470 during a deployment of the prosthetic heart valve 100. FIG. 17A depicts the deployment assist 1760 in a first or non-engaged configuration in which the deployment assist 1760 does not assist the hydraulic system 470, while FIG. 17B depicts the deployment assist 1760 in a second or engaged configuration in which the deployment assist 1760 assists the hydraulic system 470. The deployment assist 1760 includes a lever 1761 having a cam 1762 positioned within the manifold 325 and an arm 1763 which is attached to or integrally formed with the cam 1762 and is positioned externally relative to the manifold 325. The cam 1762 is coupled to the inner surface of the manifold 325 by a pin 1764, which allows for the lever 1761 to rotate relative to the manifold 325. The cam 1762 is initially positioned or disposed adjacent to the hard stop 462 but does not exert a distal force onto the hard stop 462 until the lever 1761 is rotated. When the lever 1761 is rotated about the pin 1764, due to the shape or outer perimeter configuration of the cam 1762, the cam 1762 exerts or places a distal axial force onto the hard stop 462 and thereby provides a mechanical push assist force. Due to the length of the arm 1763, the lever 1761 provides the clinician with a mechanical advantage when rotating the cam 1762 as to place a distal axial force on the hard stop 462.

More particularly, with reference to FIG. 17A, prior to use or delivery of the delivery device 320, the recapture chamber 461 is pressurized or filled with hydraulic fluid from the recapture pressure delivery device 473, moving the recapture piston 460 to the proximal most position as shown in FIG. 17A. The lever 1761 is in the first or non-engaged position in FIG. 17A, and the cam 1762 does not exert or place any axial force onto the hard stop 462.

Once the clinician inserts and navigates the delivery device 320 to the target location, the clinician may choose to provide a mechanical push assist force when deploying the capsule 322 by rotating the lever 1761 about the pin 1764. As described above, when the cam 1762 rotates, due to the shape or outer perimeter configuration of the cam 1762, the cam 1762 places or exerts an axial force onto the hard stop 462 as to drive the hard stop 462 and the capsule 322 distally. The lever 1761 is in the second or engaged position in FIG. 17B such that the cam 1762 exerts or places an axial force onto the hard stop 462.

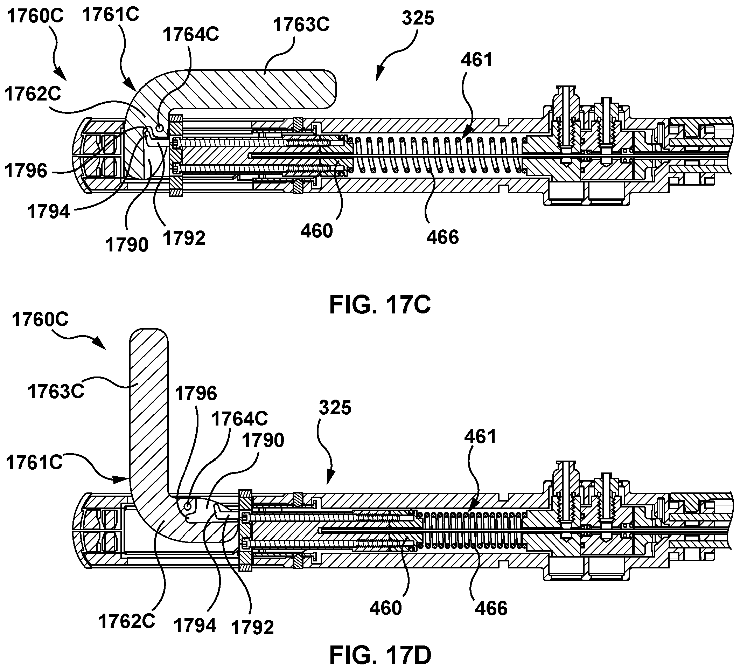

FIGS. 17C and 17D show another embodiment of a deployment assist 1760C. The deployment assist 1760C is similar to the deployment assist 1760, except that it is further configured to lock the hard stop 462 from distal movement. More particularly, the deployment assist 1760C includes a lever 1761C having a cam 1762C positioned within the manifold 325 and an arm 1763C which is attached to or integrally formed with the cam 1762C and is positioned externally relative to the manifold 325. The lever 1761C further includes a slot 1790 formed within or on the cam 1762C. The hard stop 462 includes a key or extension 1792 that is configured to be positioned within the slot 1790. The key or extension 1792 includes a radial tang 1794 at a proximal end thereof. The hard stop 462 is initially releasably coupled to the cam 1762C via a mating or interlocking relationship between the key 1792 and the slot 1790.

In a first position of the lever 1761C, the key 1792 is disposed within the slot 1790 as to restrict the hard stop 462 from moving distally, locking the hard stop 462 in the first position as shown in FIG. 17C. In the particular example of FIGS. 17C-17D, the key 1792 is positioned within the slot 1790 such that the radial tang 1794 is disposed or abuts against an internal flange 1796 of the slot 1790. Due to the interference between the radial tang 1794 and the internal flange 1796, the key 1792 is locked relative to the slot 1790 and axial movement of the key 1792, and hard stop 462 attached thereto, is prohibited. Thus, while in the first position, the lever 1761C locks the hard stop 462 from moving, and, further, the lever 1761C may be secured externally or held stationary such that no movement of the lever 1761C or the hard stop 462 is permitted. Thus, deployment of the prosthetic heart valve 100 is inhibited by the lever 1761C until the clinician selectively moves the lever 1761 out of the first position. Further, when the lever 1761C is in the first position, the cam 1762C is positioned adjacent to the hard stop 462 but does not exert a distal force onto the hard stop 462.

When the lever 1761C is rotated about the pin 1764C, the slot 1790 also moves or rotates and as a result the radial tang 1794 is no longer disposed against the internal flange 1796 of the slot 1790. Stated another way, after at least partial rotation of the cam 1762C, the slot 1790 is aligned with or oriented relative to the key 1792 as to allow the key 1792 to move distally, and, therefore, allowing the hard stop 462, and subsequently the capsule 322 and the recapture piston 460, to move distally. Thus, once at the target location, the lever 1761C can be rotated to unlock the hard stop 462 and, subsequently, the recapture piston 460 and the capsule 322 as shown in FIG. 17D. When the lever 1761C is in the second position, the key 1792 may slide distally within the slot 1790 and thus allow for the hard stop 462 to travel distally and thus the recapture piston 460 and the capsule 322 are now permitted to move axially. The clinician may then begin to use the hydraulic system 470 to distally drive the capsule 322 for deployment of the prosthetic heart valve 100.

Further, similar to the lever 1761, when the lever 1761C is rotated about the pin 1764C to transition from the first position to the second position, due to the shape or outer perimeter configuration of the cam 1762C, the cam 1762C exerts or places a distal axial force onto the hard stop 462 and thereby provides a mechanical push assist force. In another embodiment (not shown), the lever 1761A may be configured to have three positions with a first position having the key 1792 disposed within the slot 1790 to lock the hard stop 462, a second position in which the slot 1790 is moved via an initial or partial rotation of the lever 1671C such that distal movement of the key 1792 and the hard stop 462 is permitted but the cam 1762C does not yet exert or place a distal axial force onto the hard stop 462, and a third position in which the lever 1761C is further or fully rotated such that the cam 1762C exerts or places a distal axial force onto the hard stop 462 and thereby provides a mechanical push assist force.

It will be apparent to one of ordinary skill in the art that the mating geometry of the key 1792 and the slot 1790 is merely exemplary, and other configurations thereof are possible which result in the same functionality as the key 1792 and the slot 1790. In addition, it will be apparent to one of ordinary skill in the art that the profile or shape of the cam 1762, 1762C is also merely exemplary, and other configurations thereof are possible which result in the same functionality as the cam 1762, 1762C.

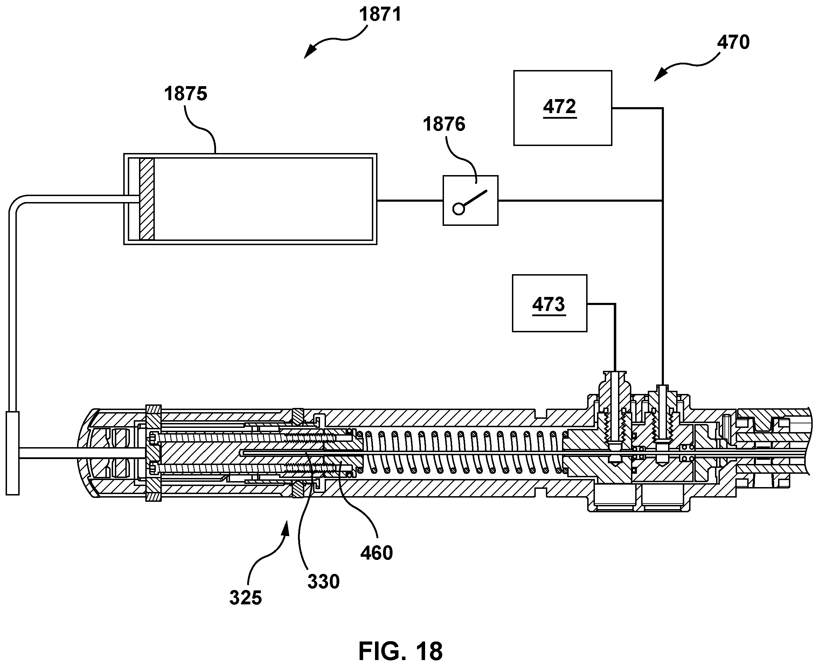

In addition to the mechanical push assists described above, hydraulic control mechanisms are also contemplated to be used in conjunction with the hydraulic system 470 to start or begin driving the capsule 322 distally. FIG. 18 is a schematic view of the delivery device 320 with a hydraulic control mechanism 1871 integrated into the hydraulic system 470 that is configured to dampen or mitigate forces during deployment of prosthetic heart valve 100. As stated above, "jumping" may occur in the delivery device 320 as a result of the hydraulic pressure being increased within the annular chamber 357 until it is high enough to exceed the required amount of force to move the capsule 322, possibly causing a rapid or unpredictable change in volume of the annular chamber 357. The hydraulic control mechanism 1871 includes a secondary or auxiliary dampening pump 1875 which delivers hydraulic fluid into the hydraulic fluid path of the delivery device 320 in order to supplement the amount or volume of hydraulic fluid delivered to the delivery device 320 by the deployment pressure delivery device 472. The secondary dampening pump 1875 functions to avoid a loss in fluid pressure within the delivery device 320 as the volume of hydraulic fluid within annular chamber 357 changes in order to reduce the risk of jumping within the delivery device 320. The secondary dampening pump 1875 is fluidly coupled to the delivery device 320 via the deployment valve 463, with a check valve 1876 positioned between the secondary dampening pump 1875 and the deployment valve 463. The check valve 1876 ensures that the hydraulic fluid is only delivered in a single direction from the secondary dampening pump 1875 into the delivery device 320.

The secondary dampening pump 1875 is further coupled to the recapture chamber 461, either at the end of a plunger extending from the recapture piston 460 as seen in FIG. 18, or may alternatively be coupled directly to the recapture piston 460 (not shown). As the recapture piston 460 moves in relation to the capsule 322, the secondary dampening pump 1875 is depressed as to insert an amount of hydraulic fluid equal to the change in volume of the annular chamber 357 in order to maintain a more consistent pressure as the volume changes. As the pressure within the delivery device 320 drops, due to movement of the deployment piston 354 as hydraulic fluid is delivered into the annular chamber 357 via the deployment pressure delivery device 472, the secondary dampening pump 1875 delivers a volume of hydraulic fluid into the delivery device 320 at a similar rate in order to keep the pressure within the delivery device 320 constant. The secondary dampening pump 1875 thus delivers hydraulic fluid to the deployment fluid path at a similar rate to the volume used to move the capsule 322 and deploy the prosthetic heart valve 100.

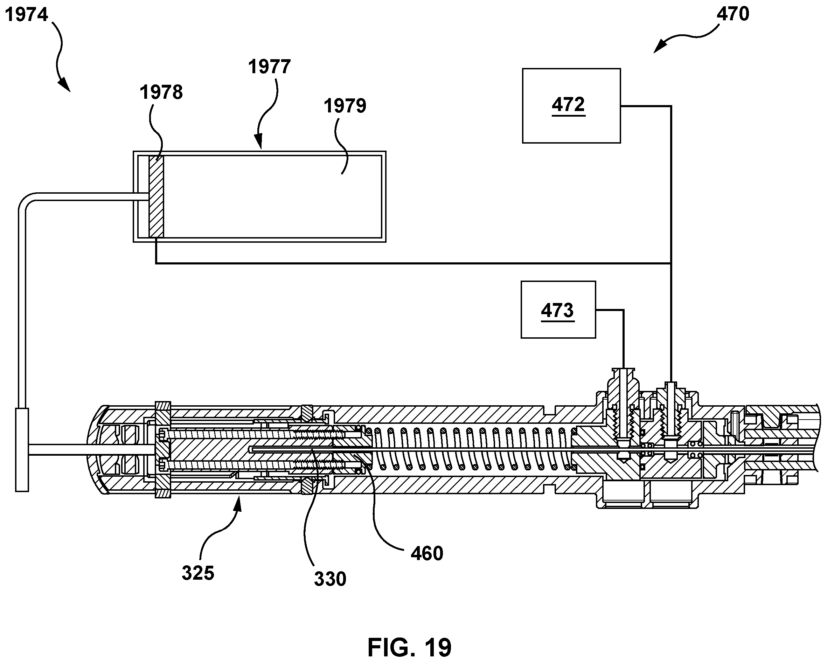

In addition to or as an alternative to the mechanical push assists described above, a hydraulic push assist is also contemplated to be used in conjunction with the hydraulic system 470 to start or begin driving the capsule 322 distally. FIG. 19 is a schematic view of the delivery device 320 with a hydraulic assist 1974. The hydraulic assist 1974 is configured to deliver supplemental hydraulic fluid to advance or push the recapture piston 460 in the distal direction, thereby driving the tension cable 330 distally to help advance the capsule 322 to deploy the prosthetic heart valve 100. As noted above, the force required to start the transition process may be greater than the force that the hydraulic system 470 is able to provide solely via the pressure within the annular chamber 357. The increased force may be due to a variety of factors, such as static friction, and the like. The hydraulic assist 1974 includes a secondary cylinder 1977 that allows for the hydraulic fluid to have a greater surface area to apply a force to transition the delivery device 320 from the delivery to the deployed configuration. The secondary cylinder 1977 includes a secondary piston 1978 and a secondary chamber 1979. The secondary chamber 1979 is fluidly coupled to the deployment pressure delivery device 472. As the amount of hydraulic fluid is increased, the volume of the annular chamber 357 increases. However, if the deployment forces are too high to drive the capsule 322 distally via hydraulic fluid in the annular chamber 357 alone, the hydraulic fluid will further be pumped into the secondary cylinder 1977, proximal to the secondary piston 1978. This hydraulic fluid will place a force on the secondary piston 1978 of the secondary cylinder 1977, causing it to move distally. The secondary piston 1978 is coupled to the recapture piston 460 such that axial movement of the secondary piston 1978 also causes axial movement of the recapture piston. So, the recapture piston 460 is driven distally with the secondary piston 1978 when hydraulic fluid is delivered into the secondary cylinder 1977. The axial movement of the recapture piston 460 places an additional mechanical force onto tension cable 330 and thereby the capsule 322, helping to overcome the initial deployment forces and thereby drive the capsule 322 distally.

In another embodiment (not shown), the secondary chamber 1979 may be included or integrated into the manifold 325 of the delivery device 320. In such an embodiment, a secondary piston 1978 is not required. Rather, the recapture piston 460 may act or function as the secondary piston, with the secondary chamber 1979 being positioned proximal to the recapture piston 460 and the recapture chamber 461 being positioned distal to the recapture piston 460.

It is further envisioned that any of the mechanical or hydraulic dampening or deployment assist solutions described herein may be used independently or in combination with one another. For example, the deployment assist 468 may be used in conjunction with the hydraulic control mechanism 1871 to limit "jumping" as well as provide an additional deployment force. Other combinations of the described embodiments are envisioned but are not described here.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A system for deploying a valve prosthesis (100) comprising:
a deployment device configured to deploy a valve prosthesis, the deployment device including:
an elongated catheter body (324A) having a distal end and a proximal end, the catheter body further including a lumen extending a length of the catheter body;
a tension cable (330) having a first end and a second end, the tension cable positioned within the catheter body;
a capsule (322) having a delivery configuration and a deployed configuration, the capsule positioned at the distal end of the catheter body and operatively coupled to the lumen and the first end of the tension cable, wherein the tension cable inhibits the capsule from transitioning between the delivery configuration and the deployed configuration;
a handle (327) positioned at the proximal end of the catheter body and operatively coupled to the second end of the tension cable and the lumen; and
a deployment assist (468) positioned at the handle and is configured to place a force on the second end of the tension cable in order to assist in transitioning the capsule from the delivery configuration to the deployed configuration;
wherein the lumen and the capsule are configured to hold a hydraulic fluid; and
a hydraulic system (470) fluidly connected to the capsule and the lumen, wherein the hydraulic system is configured to transition the capsule from the delivery configuration to the deployed configuration via delivery of the hydraulic fluid into the capsule through the lumen.

2. The system of claim 1, wherein the deployment assist is rotatable relative to the handle, wherein the rotational movement of the deployment assist places the force on the second end of the tension cable, wherein the force is an axial force in a distal direction.

3. The system of claim 1, wherein the capsule further includes a body and a deployment piston (354), wherein transitioning the capsule from the delivery configuration to the deployed configuration moves the body axially relative to the deployment piston.

4. The system of claim 3, wherein the capsule further includes a deployment chamber (357) between the body and the deployment piston and fluidly coupled to the lumen, the deployment chamber is configured to fill with hydraulic fluid to transition the capsule from the delivery configuration to the deployed configuration.

5. The system of claim 3, wherein the first end of the tension cable is coupled to the body of the capsule, the first end of the tension cable being configured to place an axial force on the body of capsule as to move the body of the capsule axially relative to the deployment piston.

6. The system of claim 1, wherein the deployment assist is a cap (1461) that is positioned radially over the proximal end of the handle, the cap is configured to have a plurality of threads (1564) that convert rotational movement of the cap into the force that is placed on the second end of the tension cable.

7. The system of claim 6, wherein the deployment assist further includes a locking aperture (1463), the locking aperture configured to receive a pin (1464) that secures the cap to the handle.

8. The system of claim 1, wherein the deployment assist (1668) is a plunger that is positioned within the proximal end of the handle, the plunger extends radially from the distal end of the handle and is configured to have a plurality of threads (1661A) that convert the rotational movement of the plunger into the force that is placed on the second end of the tension cable.

9. The system of claim 1, wherein the deployment assist (1760) further includes a lever (1761) including a cam (1762) that is configured to place the force onto the second end of the tension cable as the lever is rotated.

10. The system of claim 1, wherein the system further includes a distal push assist (735) at the capsule, the distal push assist being configured to assist in transitioning the capsule between the delivery and deployed configurations.

11. The system of claim 10, wherein the distal push assist is a spring.

12. The system of claim 1, wherein the hydraulic system (470) includes
a primary fluid cylinder (472) fluidly connected to the lumen and the capsule, the primary fluid cylinder being configured to increase the pressure of the hydraulic system to a specified pressure level; and
a secondary fluid cylinder (1977) fluidly connected to the lumen and the capsule, the secondary fluid cylinder being configured to supplement fluid volumes within the lumen and the capsule.

13. The system of claim 12, wherein the first end of the tension cable exerts a first axial force in a first direction on the capsule.

14. The system of claim 13, wherein the pressure within the lumen and the capsule exerts a second axial force in a second direction on the capsule, the second direction being opposite the first direction.

15. The system of claim 14, wherein the capsule transitions from the delivery configuration to the deployed configuration when the second axial force is larger than the first axial force; and optionally further comprising a check valve configured to control how much fluid volume is supplemented by the secondary cylinder.

## Patentansprüche

1. System zum Entfalten einer Klappenprothese (100), das Folgendes umfasst:
eine Entfaltungsvorrichtung, die konfiguriert ist, um eine Klappenprothese zu entfalten, wobei die Entfaltungsvorrichtung Folgendes einschließt:
einen länglichen Katheterkörper (324A) mit einem distalen Ende und einem proximalen Ende, wobei der Katheterkörper ferner ein Lumen einschließt, das sich über eine Länge des Katheterkörpers erstreckt;
ein Spannseil (330) mit einem ersten Ende und einem zweiten Ende, wobei das Spannseil innerhalb des Katheterkörpers positioniert ist;
eine Kapsel (322) mit einer Zuführkonfiguration und einer entfalteten Konfiguration, wobei die Kapsel an dem distalen Ende des Katheterkörpers positioniert und mit dem Lumen und dem ersten Ende des Spannseils wirkverbunden ist, wobei das Spannseil die Kapsel daran hindert, zwischen der Zuführkonfiguration und der entfalteten Konfiguration zu wechseln;
einen Griff (327), der an dem proximalen Ende des Katheterkörpers positioniert und mit dem zweiten Ende des Spannseils und dem Lumen wirkverbunden ist; und
eine Entfaltungshilfe (468), die an dem Griff positioniert und konfiguriert ist, um eine Kraft auf das zweite Ende des Spannseils auszuüben, um das Überführen der Kapsel von der Zuführkonfiguration in die entfaltete Konfiguration zu unterstützen;
wobei das Lumen und die Kapsel konfiguriert sind, um ein Hydraulikfluid zu halten; und
ein Hydrauliksystem (470), das fluidisch mit der Kapsel und dem Lumen verbunden ist, wobei das Hydrauliksystem konfiguriert ist, um die Kapsel durch Zufuhr des Hydraulikfluids durch das Lumen in die Kapsel von der Zuführkonfiguration in die entfaltete Konfiguration zu überführen.

2. System nach Anspruch 1, wobei die Entfaltungshilfe relativ zu dem Griff drehbar ist, wobei die Drehbewegung der Entfaltungshilfe die Kraft auf das zweite Ende des Spannseils ausübt, wobei die Kraft eine axiale Kraft in einer distalen Richtung ist.

3. System nach Anspruch 1, wobei die Kapsel ferner einen Körper und einen Entfaltungskolben (354) einschließt, wobei das Überführen der Kapsel von der Zuführkonfiguration in die entfaltete Konfiguration den Körper axial relativ zu dem Entfaltungskolben bewegt.

4. System nach Anspruch 3, wobei die Kapsel ferner eine Entfaltungskammer (357) zwischen dem Körper und dem Entfaltungskolben einschließt und fluidisch mit dem Lumen gekoppelt ist, wobei die Entfaltungskammer konfiguriert ist, um sich mit Hydraulikfluid zu füllen, um die Kapsel von der Zuführkonfiguration in die entfaltete Konfiguration zu überführen.

5. System nach Anspruch 3, wobei das erste Ende des Spannseils mit dem Körper der Kapsel gekoppelt ist, wobei das erste Ende des Spannseils konfiguriert ist, um eine axiale Kraft auf den Körper der Kapsel auszuüben, um den Körper der Kapsel axial relativ zu dem Entfaltungskolben zu bewegen.

6. System nach Anspruch 1, wobei die Entfaltungshilfe eine Kappe (1461) ist, die radial über dem proximalen Ende des Griffs positioniert ist, wobei die Kappe so konfiguriert ist, dass sie eine Vielzahl von Gewindegängen (1564) aufweist, die eine Rotationsbewegung der Kappe in die Kraft umwandeln, die auf das zweite Ende des Spannseils ausgeübt wird.

7. System nach Anspruch 6, wobei die Entfaltungshilfe ferner eine Verriegelungsöffnung (1463) einschließt, wobei die Verriegelungsöffnung konfiguriert ist, um einen Stift (1464) aufzunehmen, der die Kappe an dem Griff befestigt.

8. System nach Anspruch 1, wobei die Entfaltungshilfe (1668) ein Stößel ist, der innerhalb des proximalen Endes des Griffs positioniert ist, wobei sich der Stößel radial von dem distalen Ende des Griffs erstreckt und so konfiguriert ist, dass er eine Vielzahl von Gewindegängen (1661A) aufweist, die die Rotationsbewegung des Stößels in die Kraft umwandeln, die auf das zweite Ende des Spannseils ausgeübt wird.

9. System nach Anspruch 1, wobei die Entfaltungshilfe (1760) ferner einen Hebel (1761) einschließt, der eine Nocke (1762) einschließt, die konfiguriert ist, um bei Drehung des Hebels die Kraft auf das zweite Ende des Spannseils auszuüben.

10. System nach Anspruch 1, wobei das System ferner eine distale Schiebehilfe (735) an der Kapsel einschließt, wobei die distale Schiebehilfe konfiguriert ist, um das Überführen der Kapsel zwischen der Zuführ- und der entfalteten Konfiguration zu unterstützen.

11. System nach Anspruch 10, wobei die distale Schiebehilfe eine Feder ist.

12. System nach Anspruch 1, wobei das Hydrauliksystem (470) Folgendes einschließt:
einen primären Fluidzylinder (472), der fluidisch mit dem Lumen und der Kapsel verbunden ist, wobei der primäre Fluidzylinder konfiguriert ist, um den Druck des Hydrauliksystems auf ein vorgegebenes Druckniveau zu erhöhen; und
einen sekundären Fluidzylinder (1977), der fluidisch mit dem Lumen und der Kapsel verbunden ist, wobei der sekundäre Fluidzylinder konfiguriert ist, um Fluidvolumina innerhalb des Lumens und der Kapsel zu ergänzen.

13. System nach Anspruch 12, wobei das erste Ende des Spannseils eine erste axiale Kraft in einer ersten Richtung auf die Kapsel ausübt.

14. System nach Anspruch 13, wobei der Druck innerhalb des Lumens und der Kapsel eine zweite axiale Kraft in einer zweiten Richtung auf die Kapsel ausübt, wobei die zweite Richtung der ersten Richtung entgegengesetzt ist.

15. System nach Anspruch 14, wobei die Kapsel von der Zuführkonfiguration in die entfaltete Konfiguration übergeht, wenn die zweite axiale Kraft größer als die erste axiale Kraft ist; und optional ferner umfassend ein Rückschlagventil, das konfiguriert ist, um zu steuern, wie viel Fluidvolumen durch den sekundären Zylinder ergänzt wird.

## Revendications

1. Système destiné à déployer une prothèse de valve (100) comprenant :
un dispositif de déploiement conçu pour déployer une prothèse de valve, le dispositif de déploiement comportant :
un corps de cathéter allongé (324A) ayant une extrémité distale et une extrémité proximale, le corps de cathéter comportant en outre une lumière s'étendant sur une longueur du corps de cathéter ;
un câble de tension (330) ayant une première extrémité et une seconde extrémité, le câble de tension étant positionné à l'intérieur du corps de cathéter ;
une capsule (322) ayant une configuration de délivrance et une configuration déployée, la capsule étant positionnée à l'extrémité distale du corps de cathéter et accouplée de manière fonctionnelle à la lumière et à la première extrémité du câble de tension, dans lequel le câble de tension empêche la capsule d'effectuer une transition entre la configuration de délivrance et la configuration déployée ;
une poignée (327) positionnée à l'extrémité proximale du corps de cathéter et accouplée de manière fonctionnelle à la seconde extrémité du câble de tension et à la lumière ; et
un dispositif d'aide au déploiement (468) positionné au niveau de la poignée et qui est conçu pour exercer une force sur la seconde extrémité du câble de tension afin d'aider à faire passer la capsule de la configuration de pose à la configuration déployée ;
dans lequel la lumière et la capsule sont conçues pour contenir un fluide hydraulique ; et
un système hydraulique (470) relié fluidiquement à la capsule et à la lumière, dans lequel le système hydraulique est conçu pour faire passer la capsule de la configuration de pose à la configuration déployée par l'acheminement du fluide hydraulique dans la capsule à travers la lumière.

2. Système selon la revendication 1, dans lequel l'assistance au déploiement est rotative par rapport à la poignée, dans lequel le mouvement de rotation de l'assistance au déploiement exerce la force sur la seconde extrémité du câble de tension, dans lequel la force est une force axiale dans une direction distale.

3. Système selon la revendication 1, dans lequel la capsule comporte en outre un corps et un piston de déploiement (354), dans lequel le passage de la capsule de la configuration d'administration à la configuration déployée déplace le corps axialement par rapport au piston de déploiement.

4. Système selon la revendication 3, dans lequel la capsule comporte en outre une chambre de déploiement (357) entre le corps et le piston de déploiement et accouplée fluidiquement à la lumière, la chambre de déploiement est conçue pour se remplir de fluide hydraulique pour faire passer la capsule de la configuration de délivrance à la configuration déployée.

5. Système selon la revendication 3, dans lequel la première extrémité du câble de tension est couplée au corps de la capsule, la première extrémité du câble de tension étant conçue pour exercer une force axiale sur le corps de la capsule de manière à déplacer le corps de la capsule axialement par rapport au piston de déploiement.

6. Système selon la revendication 1, dans lequel l'assistance au déploiement est un capuchon (1461) qui est positionné radialement sur l'extrémité proximale de la poignée, le capuchon est conçu pour comporter une pluralité de filets (1564) qui convertissent le mouvement de rotation du capuchon en la force qui est exercée sur la seconde extrémité du câble de tension.

7. Système selon la revendication 6, dans lequel l'assistance au déploiement comporte en outre une ouverture de verrouillage (1463), l'ouverture de verrouillage étant conçue pour recevoir une goupille (1464) qui fixe le capuchon à la poignée.

8. Système selon la revendication 1, dans lequel l'assistance au déploiement (1668) est un plongeur qui est positionné à l'intérieur de l'extrémité proximale de la poignée, le plongeur s'étend radialement depuis l'extrémité distale de la poignée et est conçu pour avoir une pluralité de filets (1661A) qui convertissent le mouvement de rotation du plongeur en la force qui est exercée sur la seconde extrémité du câble de tension.

9. Système selon la revendication 1, dans lequel l'assistance au déploiement (1760) comporte en outre un levier (1761) comportant une came (1762) qui est conçue pour appliquer la force sur la seconde extrémité du câble de tension lors de la rotation du levier.

10. Système selon la revendication 1, dans lequel le système comprend en outre une assistance à la poussée distale (735) au niveau de la capsule, l'assistance à la poussée distale étant conçue pour aider à la transition de la capsule entre les configurations de délivrance et déployée.

11. Système selon la revendication 10, dans lequel l'assistance de poussée distale est un ressort.

12. Système selon la revendication 1, dans lequel le système hydraulique (470) comporte
un cylindre de fluide principal (472) relié fluidiquement à la lumière et à la capsule, le cylindre de fluide principal étant conçu pour augmenter la pression du système hydraulique à un niveau de pression spécifié ; et
un cylindre de fluide secondaire (1977) relié fluidiquement à la lumière et à la capsule, le cylindre de fluide secondaire étant conçu pour compléter les volumes de fluide à l'intérieur de la lumière et de la capsule.

13. Système selon la revendication 12, dans lequel la première extrémité du câble de tension exerce une première force axiale dans une première direction sur la capsule.

14. Système selon la revendication 13, dans lequel la pression à l'intérieur de la lumière et de la capsule exerce une seconde force axiale dans une seconde direction sur la capsule, la seconde direction étant opposée à la première direction.

15. Système selon la revendication 14, dans lequel la capsule passe de la configuration d'administration à la configuration déployée lorsque la seconde force axiale est supérieure à la première force axiale ; et comprenant en outre éventuellement un clapet anti-retour conçu pour commander le volume de fluide qui est ajouté par le cylindre secondaire.
